# EUROPEAN PATENT APPLICATION

(11) **EP 4 450 092 A1**
(43) Date of publication of application: **23.10.2024**
(21) Application number: 22907714.4
(22) Date of filing: 08.11.2022
(51) Int. Cl.: A61K 47/54, A61P 35/00

(54) **MODIFIED OLIGONUCLEOTIDE-DRUG CONJUGATE AND USE THEREOF**

(30) Priority: 16.12.2021 US 202163290155 P
(71) Applicant: Interoligo Corporation, Gyeonggi-do 14058 (KR)
(72) Inventor: LEE, Jung Hwan, Yongin-si, Gyeonggi-do 16803 (KR); LEE, Jong Ook, Seongnam-si, Gyeonggi-do 13457 (KR)
(74) Representative: dompatent von Kreisler Selting Werner - Partnerschaft von Patent- und Rechtsanwälten mbB
(86) International application number: PCT/KR2022/017440
(87) International publication number: WO 2023/113243

(57) **Abstract**

The present invention relates to a modified oligonucleotide including at least one modified nucleic acid, and a modified oligonucleotide-drug conjugate including a drug conjugated to the modified oligonucleotide. The modified oligonucleotide-drug conjugate of the present invention exhibits high stability in the body and may exhibit an excellent anticancer effect.

## Description

### [Technical Field]

The present invention relates to modified oligonucleotide-drug conjugates and a use thereof.

### [Background Art]

Currently, numerous therapeutic agents including anticancer drugs have been discovered to develop drugs through clinical trials, but continuous research is required to more selectively and effectively deliver substances with therapeutic effects, such as a targeted anticancer drug, to the desired site of symptom onset. Specifically, there is a disadvantage that the anticancer drug has a low therapeutic index and therapeutic window due to non-tumor-specific systemic toxicity and cytotoxicity, thus resistance to the anticancer drug may develop when treating for a long period of time. Accordingly, there is an urgent requirement for improved new treatments that accurately deliver drugs only to cancer cells to induce death thereof.

Meanwhile, guanosine-rich oligonucleotides may have a special structure through an intramolecular bond or intermolecular bond other than a triple hydrogen bond of guanine and cytosine. Instead of forming a double helix structure through hydrogen bonds of adenine with thymine, and guanine with cytosine, four guanines are located in one plane to form Hoogsteen hydrogen bonds, thereby resulting in a guanine tetrad (G-tetrad) with a square planar structure, and two or more guanine tetrads are stacked together to form a G-quadruplex with a quadruple helix structure. The oligonucleotides that form this G-quadruplex are known to have excellent cell permeability since they make up a stable structure due to their structural characteristics.

However, since the oligonucleotides forming the G-quadruplex should induce cell death mainly due to their cell growth inhibitory effects, the cell death rate is not relatively high, and thereby continuous treatment for more than a predetermined period of time through fluid injection for about 4 to 7 days is required. Therefore, there is a problem in that more drugs than necessary should be administered for a long period of time. Accordingly, a number of researches are being conducted to develop substances including new modified nucleic acids that are stable in the body, exhibit high cell permeability and show excellent drug efficacy.

### [Summary of Invention]

### [Problems to be Solved by Invention]

An object of the present invention is to provide modified oligonucleotide-drug conjugates.

Another object of the present invention is to provide a pharmaceutical composition for treatment or prevention of cancers, which includes modified oligonucleotide-drug conjugates.

### [Means for Solving Problems]

1. A modified oligonucleotide-drug conjugate including: a modified oligonucleotide represented by Formula 1 below; and a drug conjugated to deoxyuridine (dU) included in the modified oligonucleotide;

   [Formula 1] 5'-(M₁)ₐ-(N₁)_{b}-GGX₁GGX₂GGX₃GGX₄X₅Y₁X₆GGX₇GGX₈GGX₉GG-(N₂)_{c}-(M₂)_{d}-3'

   (in Formula 1 above,
   X₁ to X₃ and X₇ to X₉ are each independently thymidine (T), or a modified nucleic acid represented by Formula 3 or Formula 4 below;
   Yi is deoxyguanosine (dG), or a modified nucleic acid represented by Formula 3 or Formula 4 below;
   X₄ to X₆ are each independently thymidine (T), deoxyuridine (dU), or a modified nucleic acid represented by Formula 3 or Formula 4 below, and at least one of X₄ to X₆ is deoxyuridine (dU);
   M₁ and M₂ are each independently a modified nucleic acid represented by Formula 3 or Formula 4 below;
   N₁ and N₂ are each independently thymidine (T), deoxyuridine (dU), deoxycytidine (dC), or deoxyguanosine (dG);
   a and d are each independently an integer of 0 to 10, but when all of X₁ to X₉ are thymidine (T) and Yi is deoxyguanosine (dG), a and d are not 0 at the same time; and
   b and c are each independently an integer of 0 to 10),

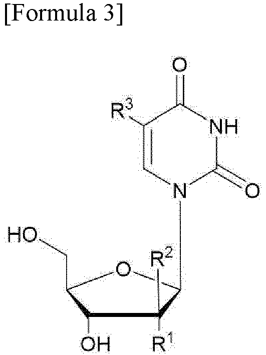

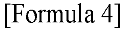

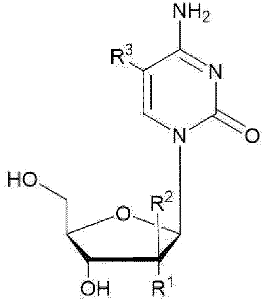

   (in Formula 3 or Formula 4 above,
   R¹ is hydrogen, halogen or a hydroxy group;
   R² is hydrogen, halogen or a hydroxy group; and
   R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).
2. The modified oligonucleotide-drug conjugate according to the above 1, wherein the modified nucleic acid represented by Formula 3 or Formula 4 above is selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluoro-2'-deoxycytidine and bromovinyldeoxyuridine.
3. The modified oligonucleotide-drug conjugate according to the above 1, wherein the drug is selected from the group consisting of paclitaxel, monomethyl auristatin E (MMAE), monomethyl auristatin F, monomethyl auristatin D, cytarabine, gemcitabine, maitansine, mertansine (DM1), DM4, calicheamicin and derivatives thereof, doxorubicin, duocarmycin and derivatives thereof, pyrrolobenzodiazepine (PBD), SN-38, α-amantine, tubulysin analog, cyclophosphamide, mecholrethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, auristatin E, auristatin F and nemorubicin.
4. The modified oligonucleotide-drug conjugate according to the above 1, wherein the drug and the deoxyuridine (dU) included in the modified oligonucleotide are conjugated by a linker L in which L₁ and L₂ are bound;
   the L₁ is selected from the group consisting of acrylamide-C2-NH₂, C12-NH₂, C3-NH₂, acrylamide-C6-propanamide-SH, acrylamide-C6-NH₂, C6-NH₂, and C6-SH, which are selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier C2 dT, amino-modifier C6 dT, S-Bz-thiol-modifier C6-dT, phosphodiester bond and nucleotide, and then obtained through a deprotection process; and
   the L₂ is selected from the group consisting of maleimidocaproyl-valine-citrulline-p-aminobenzoyloxycarbonyl (MC-Val-Cit-PAB), succinimidyl 4-(N-maleimidomethyl) cyclohexane-1-carboxylate (SMCC), succinic acid, hydrazone, peptide, disulfide, thioether, valine-citrulline, *N-*maleimidomethylcyclohexane-1-carboxylate (MCC), maleimidocaproyl, mercaptoacetamidocaproyl, *N*-succinimidyl 4-(2-pyridyldithio) pentanoate (SPP), *N*-succinimidyl 4-(2-pyridylthio) pentanoate (SPDB), phosphodiester bond and nucleotide.
5. The modified oligonucleotide-drug conjugate according to the above 1, wherein the modified oligonucleotide consists of sequences selected from SEQ ID NO: 4 to SEQ ID NO: 10.
6. A pharmaceutical composition for preventing or treating cancer including the modified oligonucleotide-drug conjugate according to any one of the above 1 to 5.
7. The pharmaceutical composition for preventing or treating cancer according to the above 6, wherein the cancer is selected from the group consisting of leukemia, lymphoma, breast cancer, liver cancer, gastric cancer, ovarian cancer, cervical carcinoma, glioma cancer, colon cancer, lung cancer, pancreatic cancer, prostate cancer, hepatoma, gastric adenocarcinoma, uterine cancer, bladder cancer, thyroid cancer, ovarian cancer, melanoma and cervical cancer.
8. A modified oligonucleotide-drug conjugate including: a modified oligonucleotide represented by Formula 2 below; and a drug conjugated to at least one of 5' and 3' ends of the modified oligonucleotide;

   [Formula 2] 5'-(M₁)ₐ-(N₁)_{b}-GGX₁GGX₂GGX₃GGX₄X₅GX₆GGX₇GGX₈GGX₉GG-(N₂)_{c}-(M₂)_{d}-3'

   (in Formula 2 above,
   X₁ to X₉ are each independently thymidine (T), or a modified nucleic acid represented by Formula 3 or Formula 4 below;
   M₁ and M₂ are each independently a modified nucleic acid represented by Formula 3 or Formula 4 below;
   N₁ and N₂ are each independently thymidine (T), deoxyuridine (dU), deoxycytidine (dC), or deoxyguanosine (dG);
   a and d are each independently an integer of 0 to 10, but when all of X₁ to X₉ are thymidine (T), a and d are not 0 at the same time; and
   b and c are each independently an integer of 0 to 10),

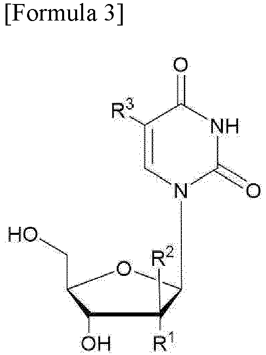

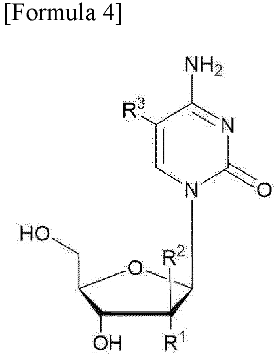

   (in Formula 3 or Formula 4 above,
   R¹ is hydrogen, halogen or a hydroxy group;
   R² is hydrogen, halogen or a hydroxy group; and
   R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).
9. The modified oligonucleotide-drug conjugate according to the above 8, wherein the modified nucleic acid represented by Formula 3 or Formula 4 above is selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluoro-2'-deoxycytidine and bromovinyldeoxyuridine.
10. The modified oligonucleotide-drug conjugate according to the above 8, wherein the drug is selected from the group consisting of paclitaxel, monomethyl auristatin E, monomethyl auristatin F, monomethyl auristatin D (MMAD), cytarabine, gemcitabine, maitansine, mertansine (DM1), DM4, calicheamicin and derivatives thereof, doxorubicin, duocarmycin and derivatives thereof, pyrrolobenzodiazepine (PBD), SN-38, α-amantine, tubulysin analog, cyclophosphamide, mecholrethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, auristatin E, auristatin F and nemorubicin.
11. The modified oligonucleotide-drug conjugate according to the above 8, wherein the drug and at least one of the 5' and 3' ends of the modified oligonucleotide represented by Formula 2 above are conjugated by a linker L in which L₁ and L₂ are bound;
   the L₁ is selected from the group consisting of (acrylamide)-C2-NH₂, C12-NH₂, C3-NH₂, acrylamide-C6-propanamide-SH, acrylamide-C6-NH₂, C6-NH₂, and C6-SH, which are selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier C2 dT, amino-modifier C6 dT, S-Bz-thiol-modifier C6-dT, phosphodiester bond and nucleotide, and then obtained through a deprotection process; and
   the L₂ is selected from the group consisting of maleimidocaproyl-valine-citrulline-p-aminobenzoyloxycarbonyl (MC-Val-Cit-PAB), succinimidyl 4-(*N-*maleimidomethyl) cyclohexane-1-carboxylate (SMCC), succinic acid, hydrazone, peptide, disulfide, thioether, valine-citrulline, *N*-maleimidomethylcyclohexane-1-carboxylate (MCC), maleimidocaproyl, mercaptoacetamidocaproyl, *N*-succinimidyl 4-(2-pyridyldithio) pentanoate (SPP), *N*-succinimidyl 4-(2-pyridylthio) pentanoate (SPDB), phosphodiester bond and nucleotide.
12. The modified oligonucleotide-drug conjugate according to the above 8, wherein the modified oligonucleotide consists of a sequence selected from SEQ ID NO: 11 or SEQ ID NO: 12.
13. A pharmaceutical composition for preventing or treating cancer including the modified oligonucleotide-drug conjugate according to any one of the above 8 to 12.

### [Advantageous effects]

The `modified oligonucleotide-drug conjugate' of the present invention includes oligonucleotide including modified nucleic acids (hereinafter referred to as `modified oligonucleotide') and a drug conjugated thereto, such that it is possible to exhibit excellent anticancer effects while showing high stability in the body.

The `modified oligonucleotide-drug conjugate' of the present invention may exhibit superior anticancer effects than the case of using the `modified oligonucleotide' alone or administering the 'drug' alone.

The `modified oligonucleotide-drug conjugate' of the present invention has a higher half-life in the body than the `modified oligonucleotide' of the same amount as the modified oligonucleotide included in the conjugate. In addition, the 'modified oligonucleotide-drug conjugate' of the present invention has lower toxicity than the 'drug' of the same amount as the drug included in the conjugate.

The `oligonucleotide modified-drug conjugate' of the present invention may exhibit superior anticancer effects than the 'oligonucleotide-drug conjugate' in which only the drug is conjugated to oligonucleotide which does not include the modified nucleic acid.

### [Brief Description of Drawings]

FIGS. 1 to 5 illustrate results of evaluating the effectiveness of modified oligonucleotide-drug conjugates in an orthotopic pancreatic cancer animal model through IVIS imaging (Specifically, FIG. 1: untreated, FIG. 2: gemcitabine-loading collagen patch treated group, FIG. 3: IO101L-loading collagen patch treated group, FIG. 4: IO176-loading collagen patch treated group, and FIG. 5: IO142-loading collagen patch treated group).

FIG. 6 illustrates results of confirming changes in the body weight of an orthotopic pancreatic cancer animal model treated with the modified oligonucleotide-drug conjugate loading collagen patch.

FIG. 7A to 7E illustrate results of confirming sizes of organs and tumors in the orthotopic pancreatic cancer animal models treated with the modified oligonucleotide-drug conjugate loading collagen patch (Specifically, FIG. 7A: untreated, FIG. 7B: gemcitabine-loading collagen patch treated group, FIG. 7C: IO101L-loading collagen patch treated group, FIG. 7D: IO176-loading collagen patch treated group, and FIG. 7E: IO142-loading collagen patch treated group)

FIG. 8A and FIG. 8B illustrate results of confirming weights of organs and tumors in the orthotopic pancreatic cancer animal models treated with the modified oligonucleotide-drug conjugate loading collagen patch.

FIG. 9 is a graph illustrating plasma concentrations of MMAE after administration of a modified oligonucleotide-drug conjugate (IO176) and MMAE according to an embodiment, wherein a small graph included in the graph shown in FIG. 9 illustrates the plasma concentration of MMAE from immediately before drug administration (0 minutes) to 120 minutes.

FIG. 10 is a graph illustrating the plasma concentrations of the modified oligonucleotide-drug conjugate (IO176), and gemcitabine and metabolite (dFdU) thereof according to an embodiment.

FIG. 11 is a scheme illustrating a process of conjugating MMAE with a modified oligonucleotide in which internal nucleotides, not ends, are replaced with modified nucleic acids.

FIG. 12 is a scheme illustrating a process of conjugating paclitaxel with the modified oligonucleotide in which internal nucleotides, not ends, are replaced with modified nucleic acids.

FIG. 13 is a scheme illustrating a process of conjugating DM1 with the modified oligonucleotide in which internal nucleotides, not ends, are replaced with modified nucleic acids.

FIG. 14 is a scheme illustrating a process of conjugating MMAE with a modified oligonucleotide in which a modified nucleic acid is added to one of 5' and 3' ends.

FIG. 15 is a scheme illustrating a process of conjugating paclitaxel with the modified oligonucleotide in which a modified nucleic acid is added to one of 5' and 3' ends.

FIG. 16 is a scheme illustrating a process of conjugating DM1 with the modified oligonucleotide in which a modified nucleic acid is added to one of 5' and 3' ends.

FIG. 17 illustrates a scheme for preparing Comparative Example 2.

FIG. 18 illustrates a scheme for preparing Example 8.

FIGS. 19 to 27 are graphs illustrating results of IC₅₀ evaluation for several examples and comparative examples.

### [Mode for Carrying out Invention]

Hereinafter, the present invention will be described in more detail.

The present invention provides a modified oligonucleotide-drug conjugate which includes a modified oligonucleotide including at least one modified nucleic acid; and a drug conjugated to the modified oligonucleotide.

### Modified oligonucleotide

The modified oligonucleotide may include at least one modified nucleic acid.

The modified oligonucleotide may include the modified nucleic acid to form a G-quadruplex structure.

Guanosine (G) contributing to the G-quadruplex structure of an oligonucleotide may be one or two or more types selected from 2'-deoxy-guanosine, guanosines, 2'-O-methyl-guanosine, 2'-fluoro-guanosine (2'-F-guanosine), locked nucleic acid (LNA)-guanosine, D-deoxyguanosine, and D-guanosine, and may be synthesized so that the modified nucleic acids with therapeutic effects are included therein.

The modified oligonucleotide may be prepared so that the above-described modified nucleic acids can be included in a guanosine-rich oligonucleotide through stationary phase synthesis using a DNA synthesizer.

The modified oligonucleotides may be prepared by replacing predetermined nucleotides in a known oligonucleotide sequence with the modified nucleic acids, or by adding and introducing the modified nucleic acid to at least one of 3' and 5' ends of the known oligonucleotide sequence.

The modified oligonucleotide may be an aptamer which has a binding ability or specificity to a target protein.

The sequence of the modified oligonucleotide and the position of the modified nucleic acid may affect the binding ability or specificity to the target protein. Therefore, the sequence thereof and the position of the modified nucleic acid should be appropriately selected in consideration of the binding ability or specificity to the target protein.

Usability of the modified oligonucleotide-drug conjugate of the present invention may be determined by the physiological activity effect that appears depending on factors such as the binding ability or specificity to the target protein.

The modified nucleic acid may have therapeutic efficacy, and may include, for example, guanosine, thymidine, cytidine or uridine with a modified sugar or base, but it is not limited thereto. The modified nucleic acid may be a pyrimidine-based nucleic acid. Specifically, the modified nucleic acid may be derived from uridine or cytidine.

The modified nucleic acid may be selected from a compound represented by Formula 3 or Formula 4 below: 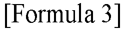 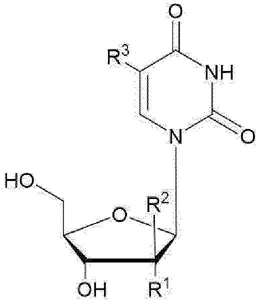 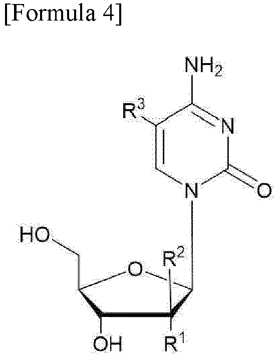
(in Formula 3 or Formula 4 above,
R¹ is hydrogen, halogen or a hydroxy group,
R² is hydrogen, halogen or a hydroxy group,
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

In Formula 3, R¹ may be hydrogen, halogen or a hydroxy group, and may specifically be hydrogen.

In Formula 3, R² may be hydrogen, halogen or a hydroxy group, and may specifically be hydrogen.

In Formula 3, R³ may be hydrogen, halogen, a C1-C6 alkyl group, a C1-C6 haloalkyl group, a C2-C6 alkenyl group or C2-C6 haloalkenyl group, and may specifically be halogen, a C1-C6 halo group or C2-C6 haloalkenyl group. According to an embodiment, Formula 3 may be 5-fluorodeoxyuridine.

In Formula 4, R¹ may be hydrogen, halogen or a hydroxy group, and may be specifically hydrogen or halogen (e.g., fluoro, chloro, bromo or iodo).

In Formula 4, R² may be hydrogen, halogen or a hydroxy group, and may be specifically hydrogen or halogen (e.g., fluoro, chloro, bromo or iodo).

In Formula 4, R³ may be hydrogen, halogen, a C1-C6 alkyl group, a C1-C6 haloalkyl group, a C2-C6 alkenyl group or C2-C6 haloalkenyl group, and may be specifically hydrogen or a C1-C6 alkyl group.

In Formula 4, R¹ and R² may be halogen, and R³ may be hydrogen or a C1 to C3 alkyl group. According to an embodiment, Formula 4 may be gemcitabine.

The modified nucleic acid may be included in the modified oligonucleotide in the form in which a phosphate group is bound to the compound represented by Formula 3 or Formula 4 above. Specifically, the modified nucleic acid may be included in the modified oligonucleotide in the form of phosphoramidite. Nucleotide phosphoramidite may be purchased from Glen Research, Berry and Associates, Okeanos Tech, Chemgene, Proligo, etc., or may be prepared by known methods (Oligonucleotides and Analogues: A Practical Approach, Fritz Eckstein et al. 1991, IRL Press: Oxford).

The modified nucleic acid may be selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluoro-2'-deoxycytidine and bromovinyldeoxyuridine. The modified nucleic acid represented by Formula 3 or Formula 4 may be selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluoro-2'-deoxycytidine and bromovinyldeoxyuridine. The 2',2'-difluoro-2'-deoxycytidine may be gemcitabine.

For example, the modified oligonucleotide may consist of a sequence represented by Formula 1 below:

[Formula 1] (M₁)ₐ-(N₁)_{b}-GGX₁GGX₂GGX₃GGX₄X₅Y₁X₆GGX₇GGX₈GGX₉GG-(N₂)_{c}-(M₂)_{d}.

The above Formula 1 is represented in a 5' to 3' direction from the left.

In the present specification, G represented in Formula 1 may be deoxyguanosine (dG).

In Formula 1 above, X₁ to X₃ and X₇ to X₉ may be each independently thymidine (T) or the modified nucleic acid represented by Formula 3 or Formula 4 described above.

In Formula 1 above, Yi may be deoxyguanosine (dG), or the modified nucleic acid represented by Formula 3 or Formula 4 described above.

In Formula 1 above, X₄ to X₆ may be each independently thymidine (T), deoxyuridine (dU), or the modified nucleic acid represented by Formula 3 or Formula 4 described above.

In Formula 1 above, at least one of X₄ to X₆ may be deoxyuridine (dU).

In Formula 1 above, M₁ and M₂ may be each independently the modified nucleic acid represented by Formula 3 or Formula 4 described above.

In Formula 1 above, N₁ and N₂ may be each independently thymidine (T), deoxyuridine (dU), deoxycytidine (dC), or deoxyguanosine (dG).

In Formula 1 above, a and d may be each independently an integer of 0 to 10.

In Formula 1 above, when all of X₁ to X₉ are thymidine (T) and Y1 is deoxyguanosine (dG), a and d may not be 0 at the same time. That is, the modified oligonucleotide represented by Formula 1 includes at least one modified nucleic acid represented by Formula 3 or Formula 4 described above.

In Formula 1 above, b and c may be each independently an integer of 0 to 10.

A drug may be conjugated to deoxyuridine (dU) of the modified oligonucleotide consisting of Formula 1 above.

Specifically, the modified oligonucleotide of the present invention may be represented by Formula 1 below:

[Formula 1] 5'-(M₁)ₐ-(N₁)_{b}-GGX₁GGX₂GGX₃GGX₄X₅Y₁X₆GGX₇GGX₈GGX₉GG-(N₂)_{c}-(M₂)_{d}-3'

(in Formula 1 above,
X₁ to X₃ and X₇ to X₉ are each independently thymidine (T), or a modified nucleic acid represented by Formula 3 or Formula 4 below;
Yi is deoxyguanosine (dG), or a modified nucleic acid represented by Formula 3 or Formula 4 below;
X₄ to X₆ are each independently thymidine (T), deoxyuridine (dU), or a modified nucleic acid represented by Formula 3 or Formula 4 below, and at least one of X₄ to X₆ is deoxyuridine (dU);
M₁ and M₂ are each independently a modified nucleic acid represented by Formula 3 or Formula 4 below;
N₁ and N₂ are each independently thymidine (T), deoxyuridine (dU), deoxycytidine (dC), or deoxyguanosine (dG);
a and d are each independently an integer of 0 to 10, but when all of X₁ to X₉ are thymidine (T) and Yi is deoxyguanosine (dG), a and d are not 0 at the same time; and
b and c are each independently an integer of 0 to 10),

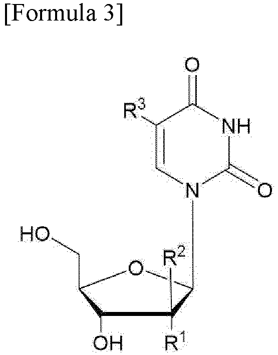

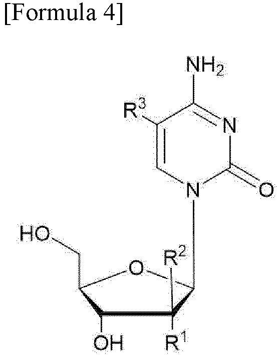

(in Formula 3 or Formula 4 above,
R¹ is hydrogen, halogen or a hydroxy group;
R² is hydrogen, halogen or a hydroxy group; and
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

According to some embodiments, the modified oligonucleotide may consist of sequences selected from SEQ ID NO: 4 to SEQ ID NO: 10.

For another example, the modified oligonucleotide may consist of a sequence represented by Formula 2 below:

[Formula 2] (M₁)ₐ-(N₁)_{b}-GGX₁GGX₂GGX₃GGX₄X₅GX₆GGX₇GGX₈GGX₉GG-(N₂)_{c}-(M₂)_{d}.

Formula 2 is represented in a 5' to 3' direction from the left.

In the present specification, G represented in Formula 2 may be deoxyguanosine (dG).

In Formula 2 above, X₁ to X₉ may be each independently thymidine (T) or the modified nucleic acid represented by Formula 3 or Formula 4 described above.

In Formula 2 above, M₁ and M₂ may be each independently the modified nucleic acid represented by Formula 3 or Formula 4 above.

In Formula 2 above, N₁ and N₂ may be each independently thymidine (T), deoxyuridine (dU), deoxycytidine (dC), or deoxyguanosine (dG).

In Formula 2 above, a and d may be each independently an integer of 0 to 10.

In Formula 2 above, when all of X₁ to X₉ are thymidine (T), a and d may not be 0 at the same time.

In Formula 2 above, b and c may be each independently an integer of 0 to 10.

A drug may be conjugated to at least one of 5' and 3' ends of the modified oligonucleotide represented by Formula 1 above.

Specifically, the modified oligonucleotide of the present invention may be represented by Formula 2 below:

[Formula 2] 5'-(M₁)ₐ-(N₁)_{b}-GGX₁GGX₂GGX₃GGX₄X₅GX₆GGX₇GGX₈GGX₉GG-(N₂)_{c}-(M₂)_{d}-3'

(in Formula 2 above,
X₁ to X₉ are each independently thymidine (T), or a modified nucleic acid represented by Formula 3 or Formula 4 below;
M₁ and M₂ are each independently a modified nucleic acid represented by Formula 3 or Formula 4 below;
N₁ and N₂ are each independently thymidine (T), deoxyuridine (dU), deoxycytidine (dC), or deoxyguanosine (dG);
a and d are each independently an integer of 0 to 10, but when all of X₁ to X₉ are thymidine (T), a and d are not 0 at the same time; and
b and c are each independently an integer of 0 to 10),

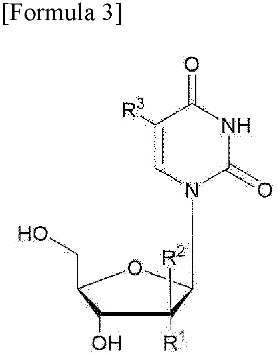

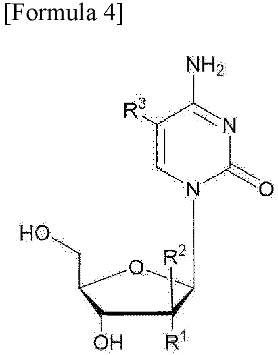

(in Formula 3 or Formula 4 above,
R¹ is hydrogen, halogen or a hydroxy group;
R² is hydrogen, halogen or a hydroxy group; and
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

According to some embodiments, the modified oligonucleotide may consist of a sequence selected from SEQ ID NO: 11 or SEQ ID NO: 12.

### Drug

The drug is sufficient as long as it is has a structure that can be conjugated to the modified oligonucleotide, and the specific type thereof is not limited.

The drug may have a functional group that can be conjugated directly to the modified oligonucleotide or a functional group that can be conjugated to a linker (e.g., a hydroxy group, a carboxyl group or amino group), but it is not limited thereto.

For example, the drug may be selected from the group consisting of paclitaxel, monomethyl auristatin E, monomethyl auristatin F, monomethyl auristatin D (MMAD), cytarabine, gemcitabine, maitansine, mertansine (DM1), DM4, calicheamicin and derivatives thereof, doxorubicin, duocarmycin and derivatives thereof, pyrrolobenzodiazepine (PBD), SN-38, α-amantine, tubulysin analog, cyclophosphamide, mecholrethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, auristatin E, auristatin F and nemorubicin.

The drug may be conjugated directly or indirectly to the modified oligonucleotide. For example, the drug may be conjugated directly to the modified oligonucleotide or conjugated through a linker.

The drug may be conjugated to at least one base included within the modified oligonucleotide. Specifically, the drug may be conjugated to uridine or deoxyuridine (dU) included within the modified oligonucleotide, but it is not limited thereto.

The drug may be conjugated to a base located at an end of the modified oligonucleotide. Specifically, the drug may be conjugated to at least one of 5' and 3' ends of the modified oligonucleotide.

The modified oligonucleotide has been described above, and therefore will not be described in detail.

### Linker

The modified oligonucleotide and the drug may be linked by a linker L. The linker L is sufficient as long as it can link the modified oligonucleotide and the drug, and the specific type thereof is not limited.

The linker L may be a linker in which L₁ and L₂ are bound.

L₁ and L₂ may be each independently conjugated to the modified oligonucleotide and the drug. L₁ and L₂ may be each independently conjugated to the modified oligonucleotide and the drug, and the modified oligonucleotide and the drug may be conjugated through a reaction with L₁ and L₂.

According to an embodiment, L₁ may be linked to the modified oligonucleotide, and L₂ may be linked to the drug. For example, L₁ may be linked to a nucleotide (e.g., dU) included within the modified oligonucleotide, and L₂ may be linked to the drug. For another example, L₁ may be linked to at least one of 5' and 3' ends of the modified oligonucleotide, and L₂ may be linked to the drug.

L₁ is sufficient as long as it includes a functional group that can be bound with L₂ through a chemical reaction, and the type thereof is not limited. L₂ is sufficient as long as it includes a functional group that can be bound with L₁ through a chemical reaction, and the type thereof is not limited.

For example, L₁ may include an -SH functional group, and L₂ may include a maleimide functional group. For another example, L₁ may include a -NH₂ functional group, and L₂ may include a -COOH functional group.

The L₁ may be selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier C2 dT, amino-modifier C6 dT, S-Bz-thiol-modifier C6-dT, phosphodiester bond and nucleotide, but it is limited thereto. L₁ may be in the deprotected form of the above-described examples, and may be (acrylamide)-C2-NH₂, C12-NH₂, C3-NH₂, acrylamide-C6-propanamide-SH, acrylamide-C6-NH₂, C6-NH₂, or C6-SH, but it is not limited thereto.

L₂ may be selected from the group consisting of maleimidocaproyl-valine-citrulline-p-aminobenzoyloxycarbonyl (MC-Val-Cit-PAB), succinimidyl 4-(N-maleimidomethyl) cyclohexane-1-carboxylate (SMCC), succinic acid, hydrazone, peptide, disulfide, thioether, valine-citrulline, *N-*maleimidomethylcyclohexane-1-carboxylate (MCC), maleimidocaproyl, mercaptoacetamidocaproyl, *N*-succinimidyl 4-(2-pyridyldithio) pentanoate (SPP), *N*-succinimidyl 4-(2-pyridylthio) pentanoate (SPDB), phosphodiester bond and nucleotide, but it is not limited thereto.

Some examples of a method for conjugating the modified oligonucleotide and the drug using the linker L are as follows.

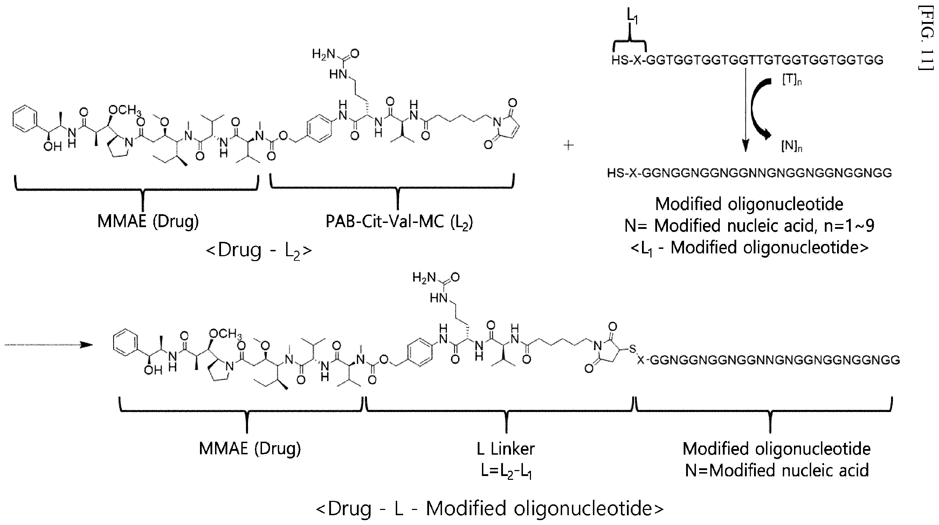

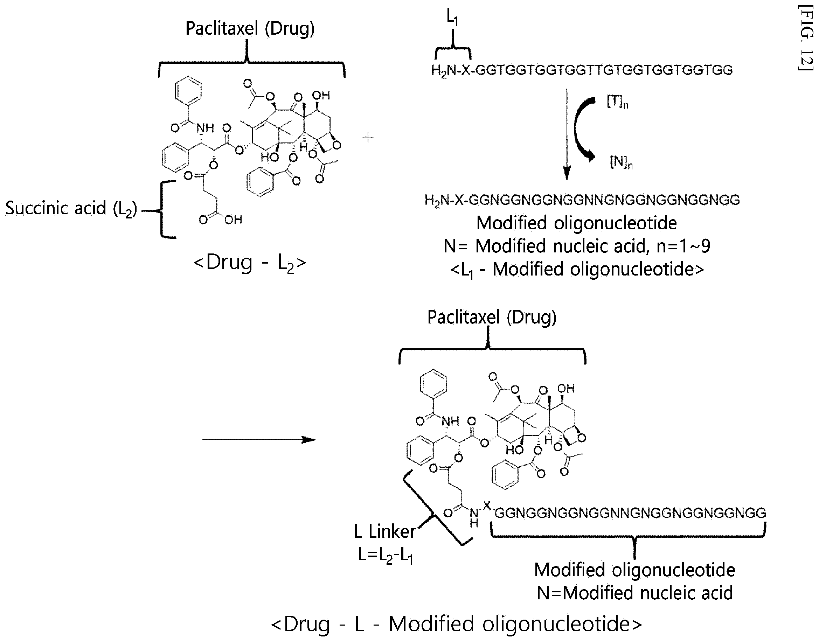

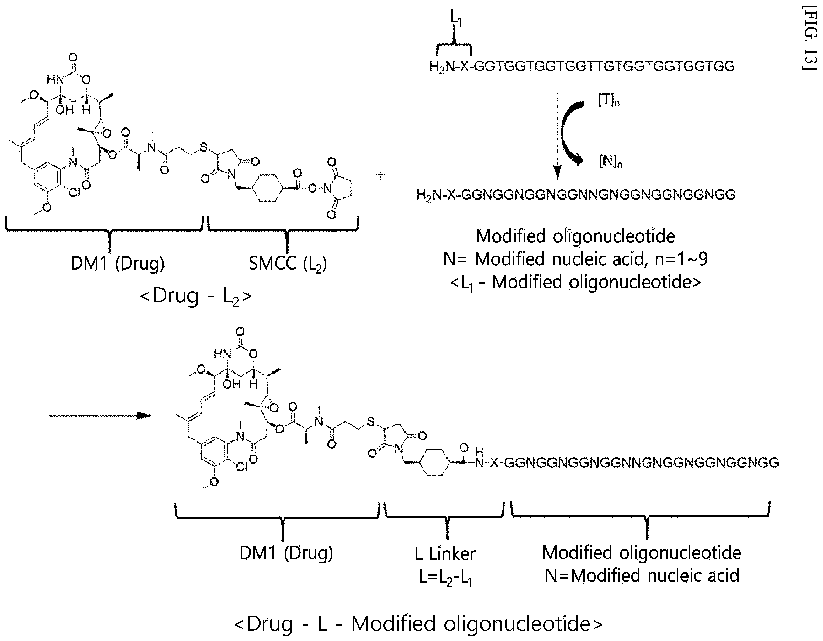

For example, a modified oligonucleotide-drug complex may be prepared by a method including the steps of: preparing a modified oligonucleotide in which predetermined nucleotides are replaced with modified nucleic acids based on a known oligonucleotide (e.g., an aptamer having the sequence of Comparative Example 1); binding L₁ to one end of the modified oligonucleotide;
binding L₂ to a drug; and
reacting the modified oligonucleotide to which L₁ is bound with the drug to which L₂ is bound (see FIGS. 11 to 13).

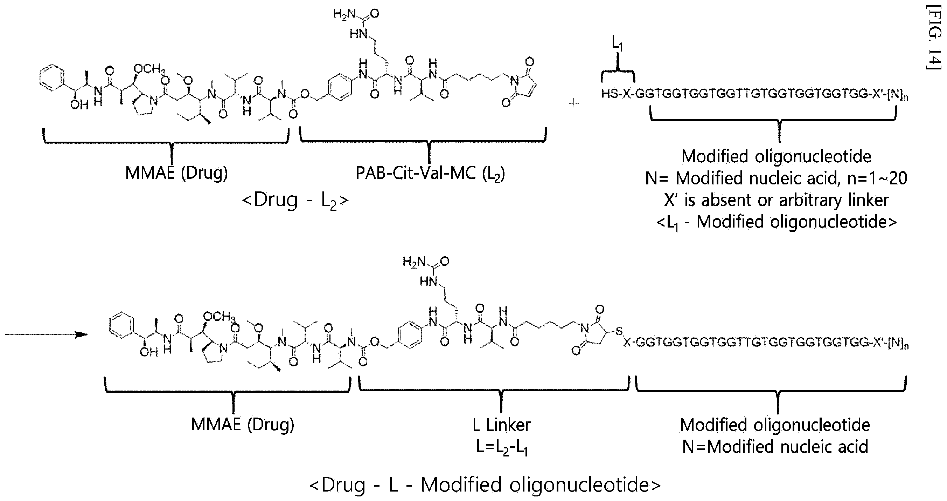

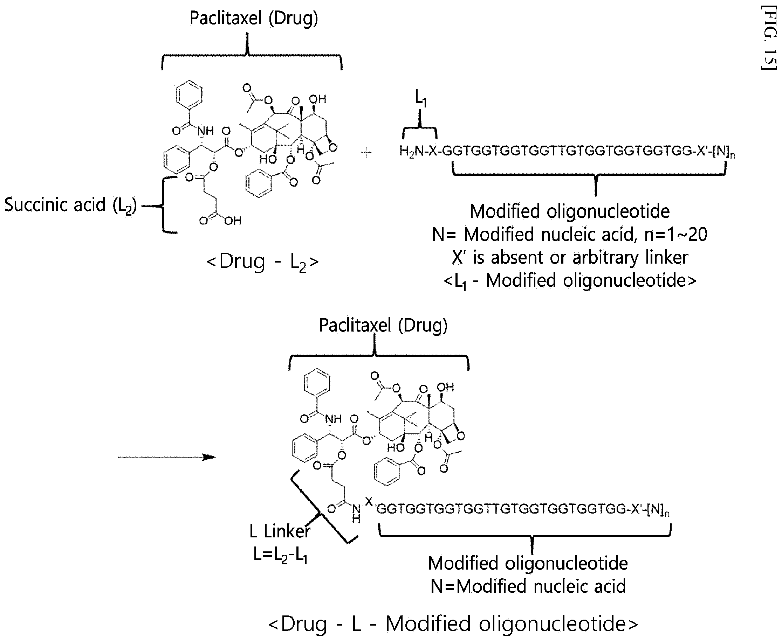

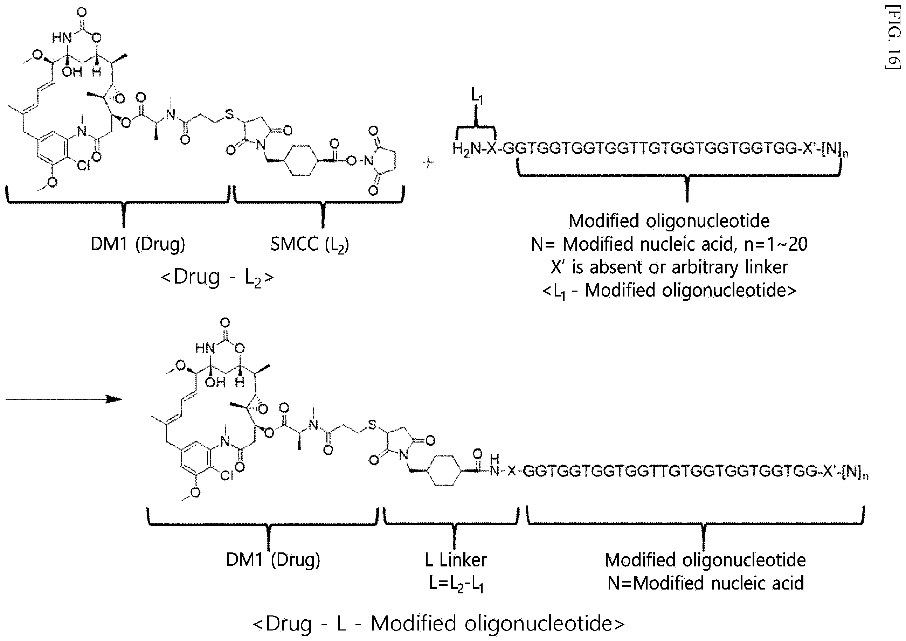

For another example, a modified oligonucleotide-drug complex may be prepared by a method including the steps of: adding at least one modified nucleic acid to at least one (5' or 3' end) of both ends of a known oligonucleotide (e.g., an aptamer having the sequence of Comparative Example 1); binding L₁ to one end of the modified oligonucleotide;
binding L₂ to a drug; and
reacting the modified oligonucleotide to which L₁ is bound with the drug to which L₂ is bound (see FIGS. 14 to 16).

According to some embodiments, the linker L may be -[acrylamide-C6-NH-(CO)-(CH₂)₂-(CO)O]-, -[C6-NH-(CO)-(CH₂)₂-(CO)O]-, -[C6-S-MC-Val-Cit-PAB]-,
-[C6-NH-CO-A]-, -[acrylamide-C6-NH-CO-A]-, or (the above A is 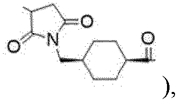 but it is not limited thereto.

### Modified oligonucleotide-drug conjugate

According to some embodiments, the modified oligonucleotide-drug conjugate of the present invention may include a modified oligonucleotide represented by Formula 1 below; and a drug conjugated to deoxyuridine (dU) included in the modified oligonucleotide:

[Formula 1] 5'-(M₁)ₐ-(N₁)_{b}-GGX₁GGX₂GGX₃GGX₄X₅Y₁X₆GGX₇GGX₈GGX₉GG-(N₂)_{c}-(M₂)_{d}-3'

(in Formula 1 above,
X₁ to X₃ and X₇ to X₉ are each independently thymidine (T), or a modified nucleic acid represented by Formula 3 or Formula 4 below;
Yi is deoxyguanosine (dG), or a modified nucleic acid represented by Formula 3 or Formula 4 below;
X₄ to X₆ are each independently thymidine (T), deoxyuridine (dU), or a modified nucleic acid represented by Formula 3 or Formula 4 below, and at least one of X₄ to X₆ is deoxyuridine (dU);
M₁ and M₂ are each independently a modified nucleic acid represented by Formula 3 or Formula 4 below;
N₁ and N₂ are each independently thymidine (T), deoxyuridine (dU), deoxycytidine (dC), or deoxyguanosine (dG);
a and d are each independently an integer of 0 to 10, but when all of X₁ to X₉ are thymidine (T) and Yi is deoxyguanosine (dG), a and d are not 0 at the same time; and
b and c are each independently an integer of 0 to 10),

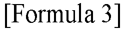

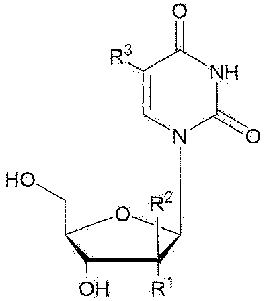

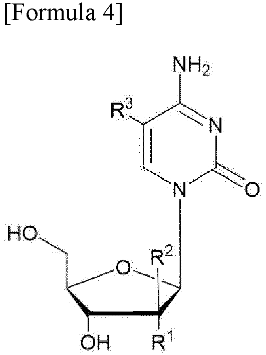

(in Formula 3 or Formula 4 above,
R¹ is hydrogen, halogen or a hydroxy group;
R² is hydrogen, halogen or a hydroxy group; and
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

The drug and the deoxyuridine (dU) may be linked by a linker L. The linker may be the above-described linker in which L₁ and L₂ are bound.

The L₁ and L₂ may be each independently bound to the deoxyuridine (dU) in the modified oligonucleotide and the drug.

Specifically, the modified oligonucleotide may consist of sequences selected from SEQ ID NO: 4 to SEQ ID NO: 10.

The modified nucleic acid, drugs, L₁ and L₂, etc. have been described above, and therefore will not be described in detail.

According to some embodiments, the modified oligonucleotide-drug conjugate of the present invention may include a modified oligonucleotide represented by Formula 2; and a drug conjugated to at least one of 5' and 3' ends of the modified oligonucleotide:

[Formula 2] 5'-(M₁)ₐ-(N₁)_{b}-GGX₁GGX₂GGX₃GGX₄X₅GX₆GGX₇GGX₈GGX₉GG-(N₂)_{c}-(M₂)_{d}-3'

(in Formula 2 above,
X₁ to X₉ are each independently thymidine (T), or a modified nucleic acid represented by Formula 3 or Formula 4 below;
M₁ and M₂ are each independently a modified nucleic acid represented by Formula 3 or Formula 4 below;
N₁ and N₂ are each independently thymidine (T), deoxyuridine (dU), deoxycytidine (dC), or deoxyguanosine (dG);
a and d are each independently an integer of 0 to 10, but when all of X₁ to X₉ are thymidine (T), a and d are not 0 at the same time; and
b and c are each independently an integer of 0 to 10),

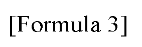

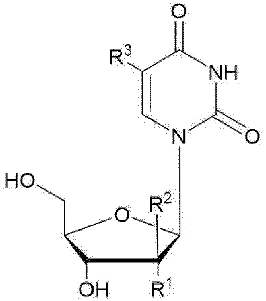

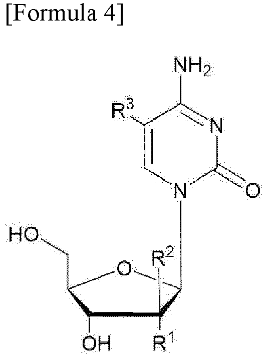

(in Formula 3 or Formula 4 above,
R¹ is hydrogen, halogen or a hydroxy group;
R² is hydrogen, halogen or a hydroxy group; and
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

At least one of 5' and 3' ends of the drug and the modified oligonucleotide may be linked by the linker L. The linker may be the above-described linker in which L₁ and L₂ are bound.

The L₁ and L₂ may be each independently bound to at least one of 5' and 3' ends of the modified oligonucleotide; and the drug.

Specifically, the modified oligonucleotide may consist of a sequence selected from SEQ ID NO: 11 or SEQ ID NO: 12.

The modified nucleic acid, drug, L₁ and L₂, etc. have been described above, and therefore will not be described in detail.

### Use of modified oligonucleotide-drug conjugate

In addition, the present invention provides a pharmaceutical composition for preventing or treating cancer including the above-described modified oligonucleotide-drug conjugate.

The cancer may be selected from the group consisting of leukemia, lymphoma, breast cancer, liver cancer, gastric cancer, ovarian cancer, cervical carcinoma, glioma cancer, colon cancer, lung cancer, pancreatic cancer, prostate cancer, hepatoma, gastric adenocarcinoma, uterine cancer, bladder cancer, thyroid cancer, ovarian cancer, melanoma and cervical cancer, but it is not limited thereto.

In addition, the present invention may provide a method for preventing or treating cancer, which includes administering the above-described modified oligonucleotide-drug conjugate to an individual.

The individual may be a mammal, including humans, and may be, for example, a human, cow, horse, dog, rabbit, cat, goat, and rat, but it is not limited thereto.

The modified oligonucleotide-drug conjugate and cancers have been described above, and therefore will not be described in detail.

Hereinafter, the configuration and effects of the present invention will be described in more detail by way of examples. However, the following examples are provided for illustrative purposes only to help understanding of the present invention, and the scope and range of the present invention are not limited thereto.

The present inventors have successfully conjugated drugs to guanosine-rich oligonucleotide variants including modified nucleic acids to synthesize modified oligonucleotide-drug conjugates, and confirmed that the synthesized modified oligonucleotide-drug conjugates are non-toxic, stable, and exhibit excellent effects of cancer treatment.

### 1. Preparation of modified oligonucleotide-drug conjugate (ApDDC; Aptamer-Double Drug-Conjugate)

Examples (modified oligonucleotide-drug conjugates) and comparative examples shown in Table 1 were designed and prepared by methods which will be described below. In Table 1 below, M denotes gemcitabine, N denotes 5-fluorodeoxyuridine (5-FdU), G is deoxyguanosine (dG), T is thymidine (T), and U is deoxyuridine (dU). That is, in the description of the present invention, G, T and U represented in SEQ ID NOs: 1 to 12 and SEQ ID NOs: 13 to 33 which are conjugates prepared using the same are deoxyguanosine (dG), thymidine (T), and deoxyuridine (dU), respectively.

**[TABLE 1]**

| **Item** | **Configuration of examples (modified oligonucleotide-drug conjugates) and comparative examples** | | | |
|---|---|---|---|---|
| | **Sequence of oligonucleotide or modified oligonucleotide (5'→3')** | **Type of conjugated drug/conjugated position** | **MW (Cal.)** | **MW (Obs.)** |
| Compara tive | GGTGGTGGTGGTTGTGGTG GTGGTGG (SEQ ID NO: 1) | - | 8272. 2750 | 8267. 3740 |
| Example 1 | | | | |
| Compara tive Example 2 | GGTGGTGGTGGTTGUGGTG GTGGTGG (SEQ ID NO: 2) | Paclitaxel/conjugated to U of SEQ ID NO: 2 (15th nucleotide of SEQ ID NO: 2) | 9362 .4482 | 9356. 7390 |
| Compara tive Example 3 | GGTGGTGGTGGTUGTGGTG GTGGTGG (SEQ ID NO: 3) | Paclitaxel/conjugated to U of SEQ ID NO: 3 (13th nucleotide of SEQ ID NO: 3) | 9362. 4482 | 9356. 8018 |
| Example 1 | GGTGGTGGTGGUMGTGGTG GTGGTGG (SEQ ID NO: 4) | Paclitaxel/conjugated to U of SEQ ID NO: 4 (12th nucleotide of SEQ ID NO: 4) | 9383. 4209 | 9377. 8057 |
| Example 2 | GGTGGTGGTGGUTMTGGTG GTGGTGG (SEQ ID NO: 5) | Paclitaxel/conjugated to U of SEQ ID NO: 5 (12th nucleotide of SEQ ID NO: 5) | 9358. 4082 | 9353. 7969 |
| Example 3 | GGTGGTGGTGGTUMTGGTG [?] GTGGTGG (SEQ ID NO: 6) | Paclitaxel/conjugated to U of SEQ ID NO: 6 (13th nucleotide of SEQ ID NO: 6) | 9358. 4082 | 9353. 7949 |
| Example 4 | GGTGGTGGTGGTUGMGGTG GTGGTGG (SEQ ID NO: 7) | Paclitaxel/conjugated to U of SEQ ID NO: 7 (13th nucleotide of SEQ ID NO: 7) | 9383. 4209 | 9377. 8047 |
| Example 5 | GGTGGTGGTGGMUGMGGT [?] GGTGGTGG (SEQ ID NO: 8) | Paclitaxel/conjugated to U of SEQ ID NO: 8 (13th nucleotide of SEQ ID NO: 8) | 9404. 3937 | 9398. 8086 |
| Example 6 | GGTGGTGGTGGTUGTGGTG GTGGMGG (SEQ ID NO: 9) | Paclitaxel/conjugated to U of SEQ ID NO: 9 (13th nucleotide of SEQ ID NO: 9) | 9383. 4209 | 9379. 8047 |
| Example 7 | GGTGGTGGTGGTTGUGGTG GTGGMGG (SEQ ID NO: 10) | Paclitaxel/conjugated to U of SEQ ID NO: 10 (15th nucleotide of SEQ ID NO: 10) | 9383. 4209 | 9377. 8076 |
| Example 8 | GGTGGTGGTGGTTGTGGTG GTGGTGGMM (SEQ ID NO: 11) | Paclitaxel/conjugated to 5'-end (i.e., 1st nucleotide) of SEQ ID NO: 11 | 10341. 9177 | 10336. 2988 |
| Compara tive Example 4 | GGTGGTGGTGGTTGTGGTG GTGGTGG (SEQ ID NO: 1) | MMAE/conjugated to 5'-end (i.e., 1st nucleotide) of SEQ ID NO: 1 | 9788. 1292 | 9782. 8164 |
| Example 9 | GGTGGTGGTGGTTGTGGTG [?] GTGGTGGMM (SEQ ID NO: 11) | MMAE/conjugated to 5'-end (i.e., 1st nucleotide) of SEQ ID NO: 11 | 10438. 4611 | 10433. 7754 |
| Compara tive Example 5 | GGTGGTGGTGGTTGTGGTG GTGGTGG (SEQ ID NO: 1) | DM1/conjugated to 5'-end (i.e., 1st nucleotide) of SEQ ID NO: 1 | 9408. 9532 | 9403. 8105 |
| Example 10 | GGTGGTGGTGGTTGTGGTG GTGGTGGMM (SEQ ID NO: 11) | DM1/conjugated to 5'-end (i.e., 1st nucleotide) of SEQ ID NO: 11 | 10059. 2852 | 10053. 0713 |
| Compara tive Example 6 | GGTGGTGGTGGTUGTGGTG GTGGTGG (SEQ ID NO: 3) | DM1/conjugated to U of SEQ ID NO: 3 (13th nucleotide of SEQ ID NO: 3) | 9384. 0088 | 9378. 8096 |
| Example 11 | GGTGGTGGTGGMUGMGGT GGTGGTGG (SEQ ID NO: 8) | DM1/conjugated to U of SEQ ID NO: 8 (13th nucleotide of SEQ ID NO: 8) | 9425. 9543 | 9402. 8115 |
| Compara tive Example 7 | GGTGGTGGTGGTTGTGGTG GTGGTGG (SEQ ID NO: 1) | Paclitaxel/conjugated to 5' of SEQ ID NO: 1 | 9387. 3926 | 9382. 1602 |
| Example 12 | GGTGGTGGTGGTTGTGGTG GTGGTGGNN (SEQ ID NO: 12) | Paclitaxel/conjugated to 5' of SEQ ID NO: 12 | 10003. 7099 | 9997. 9199 |

### (1) Preparation of Comparative Example 1

Comparative Example 1 [: GGTGGTGGTGGTTGTGGTGGTGGTGG (SEQ ID NO: 1)] was prepared by the general method for synthesis of DNA.

Specifically, oligonucleotides were synthesized using Mermade 12 or Mermade 48 (BioAutomation, USA). The oligonucleotides were sequentially synthesized through 4-step process of [Deblocking → Coupling → Capping → Oxidation] as one cycle from 3' end to 5' end by one nucleotide (1 mer). Specifically, the 4-step process of [Deblocking → Coupling → Capping → Oxidation] is as follows.
A. Deblocking: TCA Deblock was introduced to remove 4,4-dimethoxytriyl group (DMT) of nucleotide supported on a Controlled Pore Glass (CPG), thereby generating a 5'-hydroxyl group so that the next nucleotide can be bound thereto.
B. Coupling: Nucleotide phosphoramidite and ETT Activator were introduced to activate the nucleotide and bind with the 5'-hydroxyl group of the nucleotide supported on the CPG.
C. Capping: Cap A and Cap B were introduced to inactivate the 5'-hydroxyl group of (n-1) nucleotide which did not participate in the reaction.
D. Oxidation: An oxidizer was introduced to oxidize phosphate bonds between the nucleotides.

The synthesized oligonucleotides were transferred to a solution (ammonia water, 55°C) to separate the CPG support and protective groups therefrom.

Purification and analysis of the deblocked oligonucleotides were performed by the Waters Prep150 (Waters, USA) and Waters ACQUITY UPLC H-Class PLUS Bio System (Waters, USA) using a reversed-phase C18 column. In addition, mass spectrometry of purified nucleic acid ligands was performed using the Waters Xevo G2-XS Q-TOF System (Waters, USA).

### (2) Preparation of Comparative Example 2

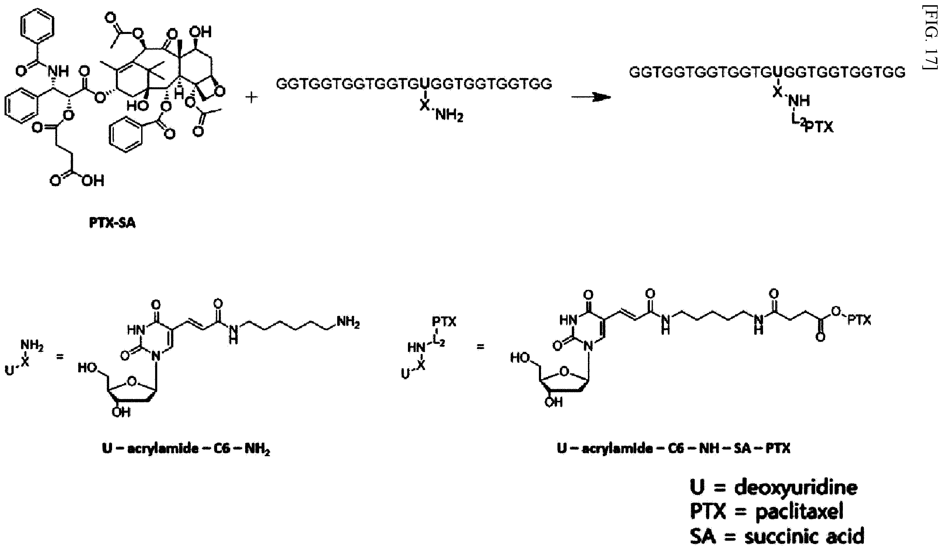

Comparative Example 2 [: Oligonucleotide-drug conjugate in which paclitaxel is conjugated to U of GGTGGTGGTGGTTGUGGTGGTGGTGG (SEQ ID NO: 2)] was prepared in two steps of the general method for synthesis of DNA and drug conjugation (see FIG. 17).

### 1) Synthesis of oligonucleotide

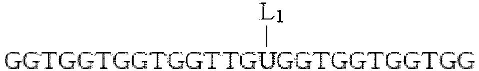

(L₁=-acrylamide-C6-NH₂) (SEQ ID NO: 13) was synthesized in the same manner as in Comparative Example 1.

### 2) Drug conjugation

Sequence of SEQ ID NO: 13 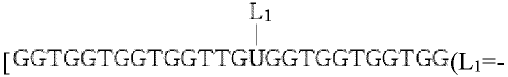 acrylamide-C6-NH₂) (SEQ ID NO: 13)] was conjugated with paclitaxel-succinic acid (PTX-SA 98%, MedKoo Biosciences, Cat#620101) to synthesize an oligonucleotide-drug conjugate of Comparative Example 2. In the oligonucleotide-drug conjugate of Comparative Example 2, the linker L linking U of the sequence of SEQ ID NO: 2 and paclitaxel is **"-acrylamide-C6-NH-succinic acid-"** (wherein acrylamide is a site linked with U of SEQ ID NO: 2, and succinic acid is a site linked with paclitaxel).

Specifically, paclitaxel-succinic acid was dissolved in DMSO, and EDC (-(3-Dimethylaminopropyl)-*N'*-ethylcarbodiimide hydrochloride) and Sulfo-NHS (*N-*hydroxysulfosuccinimide) dissolved in ultra-pure water were added thereto, followed by reacting at room temperature (RT) for 1 hour. The sequence of SEQ ID NO: 13 was dissolved in pH 8.4 buffer and the activated paclitaxel mixture was added thereto. A reaction was performed at RT for 1 hour, and the progress of reaction was observed using the Waters ACQUITY UPLC H-Class PLUS Bio System (Waters, USA).

Purification and analysis of the prepared sequence of Comparative Example 2 (oligonucleotide-drug conjugate) were performed by the Waters Prep150 (Waters, USA) and Waters ACQUITY UPLC H-Class PLUS Bio System (Waters, USA) using a reversed-phase C18 column. In addition, mass spectrometry of purified nucleic acid ligands was performed using the Waters Xevo G2-XS Q-TOF System (Waters, USA).

### (3) Preparation of Comparative Example 3

Comparative Example 3 [: Oligonucleotide-drug conjugate in which paclitaxel is conjugated to U of GGTGGTGGTGGTUGTGGTGGTGGTGG (SEQ ID NO: 3)] was prepared.

### 1) Synthesis of oligonucleotide

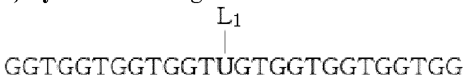

(L₁=-acrylamide-C6-NH₂) (SEQ ID NO: 14) was synthesized in the same manner as in Comparative Example 1.

### 2) Drug conjugation

The oligonucleotide-drug conjugate of Comparative Example 3 was synthesized in the same manner as in the drug conjugation method of Comparative Example 2, except for using the sequence of SEQ ID NO: 14 instead of the sequence of SEQ ID NO: 13. In the oligonucleotide-drug conjugate of Comparative Example 3, the linker L linking U of the sequence of SEQ ID NO: 3 and paclitaxel is **"-acrylamide-C6-NH-succinic acid-"** (wherein acrylamide is a site linked with U of SEQ ID NO: 3, and succinic acid is a site linked with paclitaxel).

### (4) Preparation of Example 1

Example 1 [: Modified oligonucleotide-drug conjugate in which paclitaxel is conjugated to U of GGTGGTGGTGG**UM**GTGGTGGTGGTGG (SEQ ID NO: 4), wherein M=Gemcitabine] was prepared.

### 1) Synthesis of oligonucleotide

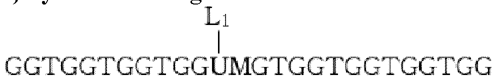

(L₁=-acrylamide-C6-NH₂, M=Gemcitabine) (SEQ ID NO: 15) was synthesized in the same manner as in Comparative Example 1.

### 2) Drug conjugation

The modified oligonucleotide-drug conjugate of Example 1 was prepared in the same drug conjugation manner as in Comparative Example 2, except for using the sequence of SEQ ID NO: 15 instead of the sequence of SEQ ID NO: 13. In the modified oligonucleotide-drug conjugate of Example 1, the linker L linking U of the sequence of SEQ ID NO: 4 and paclitaxel is **"-acrylamide-C6-NH-succinic acid-"** (wherein acrylamide is a site linked with U of SEQ ID NO: 4, and succinic acid is a site linked with paclitaxel).

### (5) Preparation of Example 2

Example 2 [: Modified oligonucleotide-drug conjugate in which paclitaxel is conjugated to U of GGTGGTGGTGG**U**T**M**TGGTGGTGGTGG (SEQ ID NO: 5), wherein M=Gemcitabine] was prepared.

### 1) Synthesis of oligonucleotide

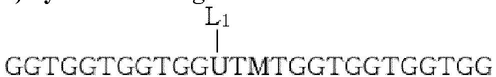

(L₁=-acrylamide-C6-NH₂, M=Gemcitabine) (SEQ ID NO: 17) was synthesized in the same manner as in Comparative Example 1.

### 2) Drug conjugation

The modified oligonucleotide-drug conjugate of Example 2 was prepared in the same drug conjugation manner as in Comparative Example 2, except for using the sequence of SEQ ID NO: 17 instead of the sequence of SEQ ID NO: 13.

In the modified oligonucleotide-drug conjugate of Example 2, the linker L linking U of SEQ ID NO: 5 and paclitaxel is **"-acrylamide-C6-NH-succinic acid-"** (wherein acrylamide is a site linked with U of SEQ ID NO: 5, and succinic acid is a site linked with paclitaxel).

### (6) Preparation of Example 3

Example 3 [: Modified oligonucleotide-drug conjugate in which paclitaxel is conjugated to U of GGTGGTGGTGGT**UM**TGGTGGTGGTGG (SEQ ID NO: 6), wherein M=Gemcitabine] was prepared.

### 1) Synthesis of oligonucleotide

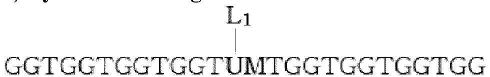

(L₁=-acrylamide-C6-NH₂, M=Gemcitabine) (SEQ ID NO: 18) was synthesized in the same manner as in Comparative Example 1.

### 2) Drug conjugation

The modified oligonucleotide-drug conjugate of Example 3 was prepared in the same drug conjugation manner as in Comparative Example 2, except for using the sequence of SEQ ID NO: 18 instead of the sequence of SEQ ID NO: 13.

In the modified oligonucleotide-drug conjugate of Example 3, the linker L linking U of the sequence of SEQ ID NO: 6 and paclitaxel is **"-acrylamide-C6-NH-succinic acid-"** (wherein acrylamide is a site linked with U of SEQ ID NO: 6, and succinic acid is a site linked with paclitaxel).

### (7) Preparation of Example 4

Example 4 [: Modified oligonucleotide-drug conjugate in which paclitaxel is conjugated to U of GGTGGTGGTGGT**U**G**M**GGTGGTGGTGG (SEQ ID NO: 7), wherein M=Gemcitabine] was prepared.

### 1) Synthesis of oligonucleotide

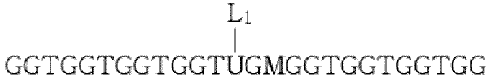

(L₁=-acrylamide-C6-NH₂, M=Gemcitabine) (SEQ ID NO: 19) was synthesized in the same manner as in Comparative Example 1.

### 2) Drug conjugation

The modified oligonucleotide-drug conjugate of Example 4 was prepared in the same drug conjugation manner as in Comparative Example 2, except for using the sequence of SEQ ID NO: 19 instead of the sequence of SEQ ID NO: 13.

In the modified oligonucleotide-drug conjugate of Example 4, the linker L linking U of the sequence of SEQ ID NO: 7 and paclitaxel is **"-acrylamide-C6-NH-succinic acid-"** (wherein acrylamide is a site linked with U of SEQ ID NO: 7, and succinic acid is a site linked with paclitaxel).

### (8) Preparation of Example 5

Example 5 [: Modified oligonucleotide-drug conjugate in which paclitaxel is conjugated to U of GGTGGTGGTGG**MU**G**M**GGTGGTGGTGG (SEQ ID NO: 8), wherein M=Gemcitabine] was prepared.

### 1) Synthesis of oligonucleotide

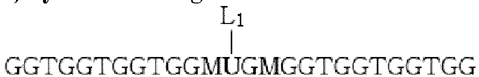

(L₁=-acrylamide-C6-NH₂, M=Gemcitabine) (SEQ ID NO: 20) was synthesized in the same manner as in Comparative Example 1.

### 2) Drug conjugation

The modified oligonucleotide-drug conjugate of Example 5 was prepared in the same drug conjugation manner as in Comparative Example 2, except for using the sequence of SEQ ID NO: 20 instead of the sequence of SEQ ID NO: 13.

In the modified oligonucleotide-drug conjugate of Example 5, the linker L linking U of the sequence of SEQ ID NO: 8 and paclitaxel is **"-acrylamide-C6-NH-succinic acid-"** (wherein acrylamide is a site linked with U of SEQ ID NO: 8, and succinic acid is a site linked with paclitaxel).

### (9) Preparation of Example 6

Example 6 [: Modified oligonucleotide-drug conjugate in which paclitaxel is conjugated to U of GGTGGTGGTGGT**U**GTGGTGGTGG**M**GG (SEQ ID NO: 9), wherein M=Gemcitabine] was prepared.

### 1) Synthesis of oligonucleotide

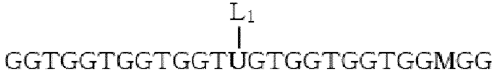

(L₁=-acrylamide-C6-NH₂, M=Gemcitabine) (SEQ ID NO: 21) was synthesized in the same manner as in Comparative Example 1.

### 2) Drug conjugation

The modified oligonucleotide-drug conjugate of Example 6 was prepared in the same drug conjugation manner as in Comparative Example 2, except for using the sequence of SEQ ID NO: 21 instead of the sequence of SEQ ID NO: 13.

In the modified oligonucleotide-drug conjugate of Example 6, the linker L linking U of the sequence of SEQ ID NO: 9 and paclitaxel is **"-acrylamide-C6-NH-succinic acid-"** (wherein acrylamide is a site linked with U of SEQ ID NO: 9, and succinic acid is a site linked with paclitaxel).

### (10) Preparation of Example 7

Example 7 [: Modified oligonucleotide-drug conjugate in which paclitaxel is conjugated to U of GGTGGTGGTGGTTG**U**GGTGGTGG**M**GG (SEQ ID NO: 10), wherein M=Gemcitabine] was prepared.

### 1) Synthesis of oligonucleotide

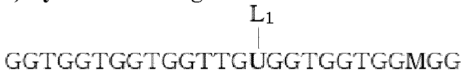

(L₁=-acrylamide-C6-NH₂, M=Gemcitabine) (SEQ ID NO: 22) was synthesized in the same manner as in Comparative Example 1.

### 2) Drug conjugation

The modified oligonucleotide-drug conjugate of Example 7 was prepared in the same drug conjugation manner as in Comparative Example 2, except for using the sequence of SEQ ID NO: 22 instead of the sequence of SEQ ID NO: 13.

In the modified oligonucleotide-drug conjugate of Example 7, the linker L linking U and paclitaxel in the sequence of SEQ ID NO: 10 is **"-acrylamide-C6-NH-succinic acid-"** (wherein acrylamide is a site linked with U of SEQ ID NO: 10, and succinic acid is a site linked with paclitaxel).

### (11) Preparation of Example 8

Example 8 [: Modified oligonucleotide-drug conjugate in which paclitaxel is conjugated to the 5' end (i.e., 1st nucleotide) of

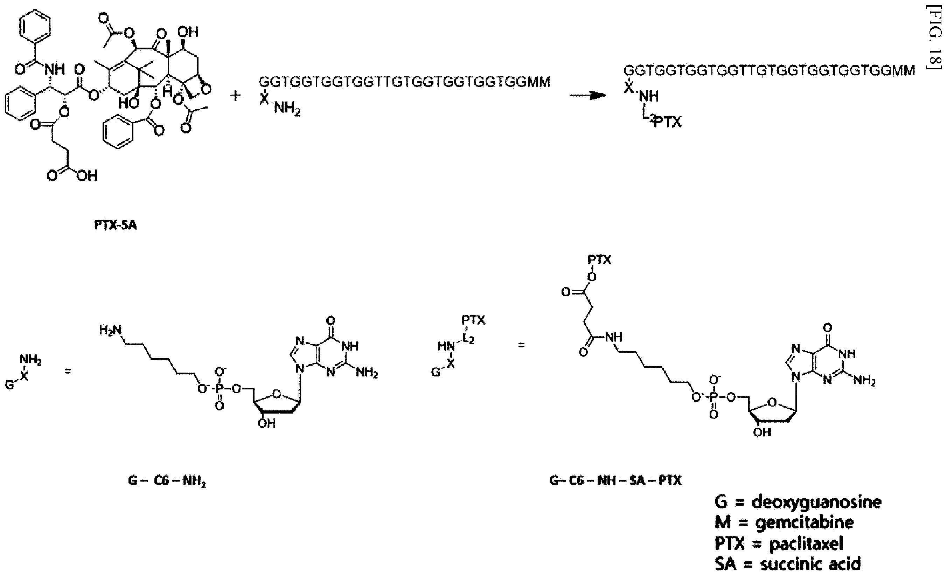

GGTGGTGGTGGTTGTGGTGGTGGTGGMM (SEQ ID NO: 11), wherein M=Gemcitabine] was prepared in the following manner (see FIG. 18).

### 1) Synthesis of oligonucleotide

L₁-GGTGGTGGTGGTTGTGGTGGTGGTGGMM(L₁=-C6-NH₂, M=Gemcitabine) (SEQ ID NO: 23) was synthesized in the same manner as in Comparative Example 1.

### 2) Drug conjugation

The modified oligonucleotide-drug conjugate of Example 8 was prepared in the same drug conjugation manner as in Comparative Example 2, except for using the sequence of SEQ ID NO: 23 instead of SEQ ID NO: 13.

In the modified oligonucleotide-drug conjugate of Example 8, the linker L linking the first nucleotide G at the 5' end of the sequence of SEQ ID NO: 11 and paclitaxel is **"-C6-NH-succinic acid-"** (wherein C6 is a site linked with the 5' end of SEQ ID NO: 11, and succinic acid is a site linked with paclitaxel).

### (12) Preparation of Comparative Example 4

Comparative Example 4 [: Oligonucleotide-drug conjugate in which MMAE is conjugated to the 5' end (i.e., 1st nucleotide) of GGTGGTGGTGGTTGTGGTGGTGGTGG (SEQ ID NO: 1)] was prepared in two steps of the general method for synthesis of DNA and drug conjugation.

### 1) Synthesis of oligonucleotide

Z-GGTGGTGGTGGTTGTGGTGGTGGTGG (Z=-C6-SS-C6-OH) (SEQ ID NO: 24) was synthesized in the same manner as in Comparative Example 1.

### 2) Drug conjugation

L₁-GGTGGTGGTGGTTGTGGTGGTGGTGG (L₁=-C6-SH) (SEQ ID NO: 25) was obtained by reducing Z of SEQ ID NO: 24 into Dithiothreitol (DTT). The oligonucleotide-drug conjugate of Comparative Example 4 was prepared by conjugating L₁ of SEQ ID NO: 25 with MC (maleimide-caproic acid)-Val-Cit-PAB (para-aminobenzyl carbamate)-MMAE (Monomethyl auristatin E) (: VcMMAE 96%, MedKoo Biosciences, Cat#407406). In the oligonucleotide-drug conjugate of Comparative Example 4, the linker L linking the first nucleotide G at the 5' end of SEQ ID NO: 1 and MMAE is **"-C6-S-MC-Val-Cit-PAB-"** (wherein C6 is a site linked with the 5' end of SEQ ID NO: 1, and PAB is a site linked with MMAE).

Specifically, DTT dissolved in pH 8.5 buffer was put into the sequence [.Z-GGTGGTGGTGGTTGTGGTGGTGGTGG(Z=-C6-SS-C6-OH)] of SEQ ID NO: 24 and reacted at RT, then centrifugation was performed at 4°C to remove remaining DTT. The activated sequence [.L₁-GGTGGTGGTGGTTGTGGTGGTGGTGG(L₁=-C6-SH)] of SEQ ID NO: 25 was dissolved in pH 6.0 buffer, then VcMMAE dissolved in DMSO was added thereto. A reaction was performed at RT for 1 hour, and the progress of reaction was observed using the Waters ACQUITY UPLC H-Class PLUS Bio System (Waters, USA).

Purification and analysis of the prepared sequence of Comparative Example 4 were performed by the Waters Prep150 (Waters, USA) and Waters ACQUITY UPLC H-Class PLUS Bio System (Waters, USA) using a reversed-phase C18 column. In addition, mass spectrometry of purified nucleic acid ligands was performed using the Waters Xevo G2-XS Q-TOF System (Waters, USA).

### (13) Preparation of Example 9

Example 9 [: Modified oligonucleotide-drug conjugate in which MMAE is conjugated to the 5' end (i.e., 1st nucleotide) of GGTGGTGGTGGTTGTGGTGGTGGTGG**MM** (SEQ ID NO: 11), wherein M=Gemcitabine] was prepared.

### 1) Synthesis of oligonucleotide

Z-GGTGGTGGTGGTTGTGGTGGTGGTGGMM(Z=-C6-SS-C6-OH, M=Gemcitabine) (SEQ ID NO: 26) was synthesized in the same manner as in Comparative Example 1.

### 2) Drug conjugation

Example 9 was prepared in the same drug conjugation manner as in Comparative Example 4, except for reducing the sequence of SEQ ID NO: 26 into DTT instead of the sequence of SEQ ID NO: 24 to obtain L₁-GGTGGTGGTGGTTGTGGTGGTGGTGGMM(L₁=-C6-SH, M=Gemcitabine) (SEQ ID NO: 27), and conjugating L₁ of SEQ ID NO: 27 with VcMMAE.

In the modified oligonucleotide-drug conjugate of Example 9, the linker L linking the first nucleotide G at the 5' end of SEQ ID NO: 11 and MMAE is **"-C6-S-MC-Val-Cit-PAB-"** (wherein C6 is a site linked with the 5' end of SEQ ID NO: 11, and PAB is a site linked with MMAE).

### (14) Preparation of Comparative Example 5

Comparative Example 5 [: Oligonucleotide-drug conjugate in which DM1 is conjugated to the 5' end (i.e., 1st nucleotide) of GGTGGTGGTGGTTGTGGTGGTGGTGG (SEQ ID NO: 1)] was prepared in two steps of the general method for synthesis of DNA and drug conjugation

### 1) Synthesis of oligonucleotide

L₁-GGTGGTGGTGGTTGTGGTGGTGGTGG (L₁=-C6-NH₂) (SEQ ID NO: 28) was synthesized in the same experimental manner as in Comparative Example 1.

### 2) Drug conjugation

Comparative Example 5 was prepared by conjugating the sequence of SEQ ID NO: 28 with Mertansin (DM1)-succinimidyl-4-(N maleimidomethyl)-cyclohexane-1-carboxylate (SMCC) (: DM1-SMCC 99%, MedKoo Biosciences, Cat#407202). In the oligonucleotide-drug conjugate of Comparative Example 5, the linker L linking the first nucleotide G at the 5' end of SEQ ID NO: 1 and DM1 is **"-C6-NH-SMCC-"** (wherein C6 is a site linked with the 5' end, and SMCC is a site linked with MMAE).

Specifically, the sequence [.L₁-GGTGGTGGTGGTTGTGGTGGTGGTGG(L₁=-C6-NH₂)] of SEQ ID NO: 28 was dissolved in pH 8.5 buffer and DM1-SMCC dissolved in DMSO was added thereto. A reaction was performed at RT for 1 hour, and the progress of reaction was observed using the Waters ACQUITY UPLC H-Class PLUS Bio System (Waters, USA). Purification and analysis of the prepared sequence of Comparative Example 5 were performed by the Waters Prep150 (Waters, USA) and Waters ACQUITY UPLC H-Class PLUS Bio System (Waters, USA) using a reversed-phase C18 column. In addition, mass spectrometry of purified nucleic acid ligands was performed using the Waters Xevo G2-XS Q-TOF System (Waters, USA).

### (15) Preparation of Example 10

Example 10 [Modified oligonucleotide-drug conjugate in which DM1 is conjugated to the 5' end (i.e., 1st nucleotide) of GGTGGTGGTGGTTGTGGTGGTGGTGG**MM** (SEQ ID NO: 11), wherein M=Gemcitabine] was prepared.

### 1) Synthesis of oligonucleotide

L₁-GGTGGTGGTGGTTGTGGTGGTGGTGGMM (L₁=-C6-NH₂, M=Gemcitabine) (SEQ ID NO: 29) was synthesized in the same experimental manner as in Comparative Example 1.

### 2) Drug conjugation

Example 10 was prepared in the same drug conjugation manner as in Comparative Example 5, except for conjugating DM1-SMCC with the sequence of SEQ ID NO: 29 instead of the sequence of SEQ ID NO: 28.

In the modified oligonucleotide-drug conjugate of Example 10, the linker L linking the first nucleotide G of the 5' end of SEQ ID NO: 11 and DM1 is **"-C6-NH-SMCC-"** (wherein C6 is a site linked with the 5' end of SEQ ID NO: 11, and SMCC is a site linked with DM1).

### (16) Preparation of Comparative Example 6

Comparative Example 6 [: Oligonucleotide-drug conjugate in which DM1 is conjugated to U of GGTGGTGGTGGT**U**GTGGTGGTGGTGG (SEQ ID NO: 3)] was prepared.

### 1) Synthesis of oligonucleotide

GGTGGTGGTGGTUGTGGTGGTGGTGG (L₁=-acrylamide-C6-NH₂) (SEQ ID NO: 30) was synthesized in the same experimental manner as in Comparative Example 1.

### 2) Drug conjugation

Comparative Example 6 was prepared in the same drug conjugation manner as in Comparative Example 5, except for conjugating DM1-SMCC with the sequence of SEQ ID NO: 30 instead of the sequence of SEQ ID NO: 28. In the oligonucleotide-drug conjugate of Comparative Example 6, the linker L linking U of SEQ ID NO: 3 and DM1 is **"-acrylamide-C6-NH-SMCC-"** (wherein acrylamide is a site linked with U of SEQ ID NO: 3, and SMCC is a site linked with DM1).

### (17) Preparation of Example 11

Example 11 [: Modified oligonucleotide-drug conjugate in which DM1 is conjugated to U of GGTGGTGGTGG**MU**G**M**GGTGGTGGTGG (SEQ ID NO: 8), wherein M=Gemcitabine] was prepared.

### 1) Synthesis of oligonucleotide

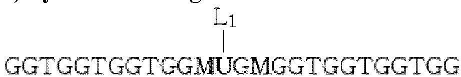

(L₁=-acrylamide-C6-NH₂, M=Gemcitabine) (SEQ ID NO: 31) was synthesized in the same experimental manner as in Comparative Example 1.

### 2) Drug conjugation

Example 11 was prepared in the same drug conjugation manner as in Comparative Example 5, except for conjugating DM1-SMCC with the sequence of SEQ ID NO: 31 instead of the sequence of SEQ ID NO: 28. In the modified oligonucleotide-drug conjugate of Example 11, the linker L linking U of 9 and DM1 is **"-acrylamide-C6-NH-SMCC-"** (wherein acrylamide is a site linked with U of SEQ ID NO: 8, and SMCC is a site linked with DM1).

### (18) Preparation of Comparative Example 7

Comparative Example 7 [: Oligonucleotide-drug conjugate in which paclitaxel is conjugated to the 5' end of GGTGGTGGTGGTTGTGGTGGTGGTGG (SEQ ID NO: 1)] was prepared.

### 1) Synthesis of oligonucleotide

L₁-GGTGGTGGTGGTTGTGGTGGTGGTGG (L₁=-C6-NH₂) (SEQ ID NO: 32) was synthesized in the same manner as in Comparative Example 1.

### 2) Drug conjugation

Comparative Example 7 was prepared in the same drug conjugation manner as in Comparative Example 2, except for using the sequence of SEQ ID NO: 32 instead of SEQ ID NO: 13.

In the oligonucleotide-drug conjugate of Comparative Example 7, the linker L linking the first nucleotide G at the 5' end of the sequence of SEQ ID NO: 1 and paclitaxel is **"-C6-NH-succinic acid-"** (wherein C6 is a site linked with the 5' end of number 1, and succinic acid is a site linked with paclitaxel).

### (19) Preparation of Example 12

Example 12 [Modified oligonucleotide-drug conjugate in which paclitaxel is conjugated to the 5' end of GGTGGTGGTGGTTGTGGTGGTGGTGG**NN** (SEQ ID NO: 12), where N=5-FdU] was prepared.

### 1) Synthesis of oligonucleotide

L₁-GGTGGTGGTGGTTGTGGTGGTGGTGGNN (L₁=-C6-NH₂, N=5-FdU) (SEQ ID NO: 33) was synthesized in the same manner as in Comparative Example 1.

### 2) Drug conjugation

Example 12 was prepared in the same drug conjugation manner as in Comparative Example 2, except for using the sequence of SEQ ID NO: 33 instead of SEQ ID NO: 13.

In the modified oligonucleotide-drug conjugate of Example 12, the linker L linking the first nucleotide G at the 5' end of the sequence of SEQ ID NO: 12 and paclitaxel is **"-C6-NH-succinic acid-"** (wherein C6 is a site linked with the 5' end of SEQ ID NO: 12, and succinic acid is a site linked with paclitaxel).

### 2. Confirmation of anticancer efficacy of modified oligonucleotide-drug conjugates - in vitro test

The *in vitro* anticancer efficacy of the modified oligonucleotide-drug conjugates was verified using pancreatic cancer cell lines. Pancreatic cancer cell lines (BxPC-3) were treated with the above-described examples and comparative examples at concentrations of 0, 0.01, 0.1, 1, 10, 100, 500 and 1000 µM, and the specific IC₅₀ measurement method and measurement results are as follows.

### (1) Cell culture

All cell lines were cultured in a 5% CO₂ incubator at 37°C. The BxPC-3 pancreatic cancer cell lines were cultured using a medium containing 10% FBS and 1% antibiotics put into ATCC modified RPMI-1640 (Thermo Scientific, USA). The medium was replaced once every 2-3 days, and passage-cultured at a time when about 70-90% of a culture dish was filled with the cells. First, the cells were washed with PBS, then 0.05% Trypsin-EDTA was added and cultured at 37°C for 2 minutes, and then a new medium was added to inactivate trypsin. The cells were separated from trypsin and medium using a centrifuge, then another new medium was added and passaged at a ratio of 1:4, followed by culturing the same. All cells were periodically checked for contamination by mycoplasma using a kit (Intron, Korea), and in vitro efficacy evaluation experiments were conducted using only cells in which contamination was not detected.

### (2) Confirmation of pancreatic cancer cell growth inhibitory effect

Using WST reagent (Dongin LS, Korea), an inhibitory effect on pancreatic cancer cell growth by drug treatment was confirmed and IC₅₀ was measured. An orange coloring substance called formazan is produced in WST by mitochondrial dehydrogenase within cells, which is effective only in living cells. The specific test method is as follows.

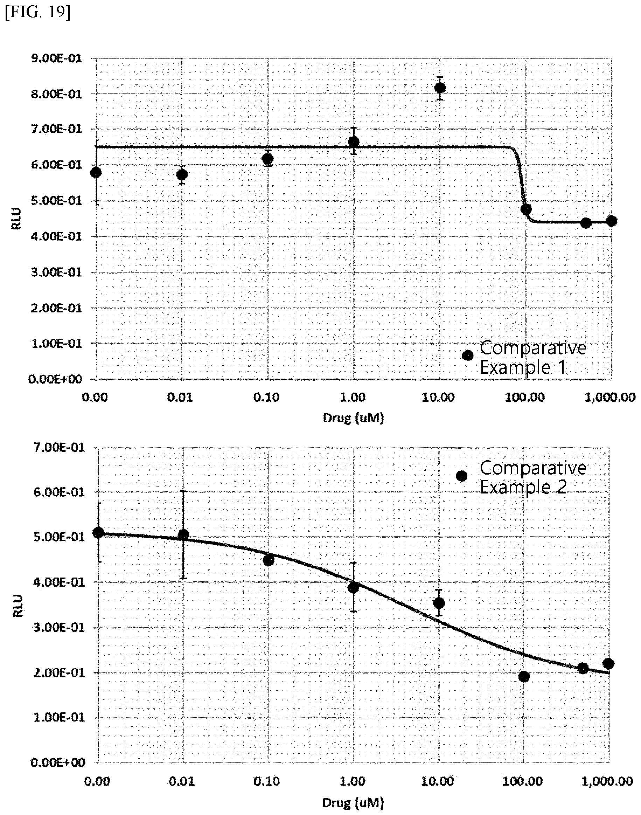

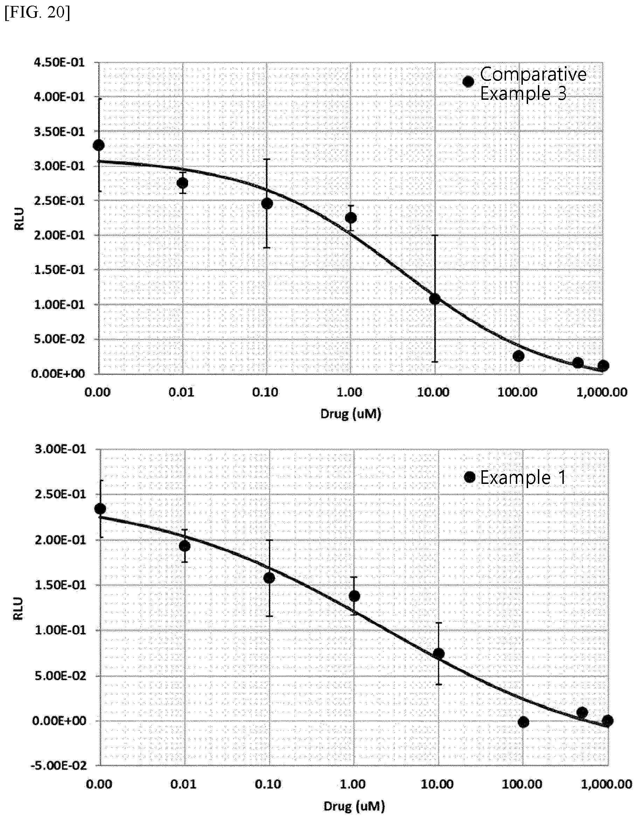

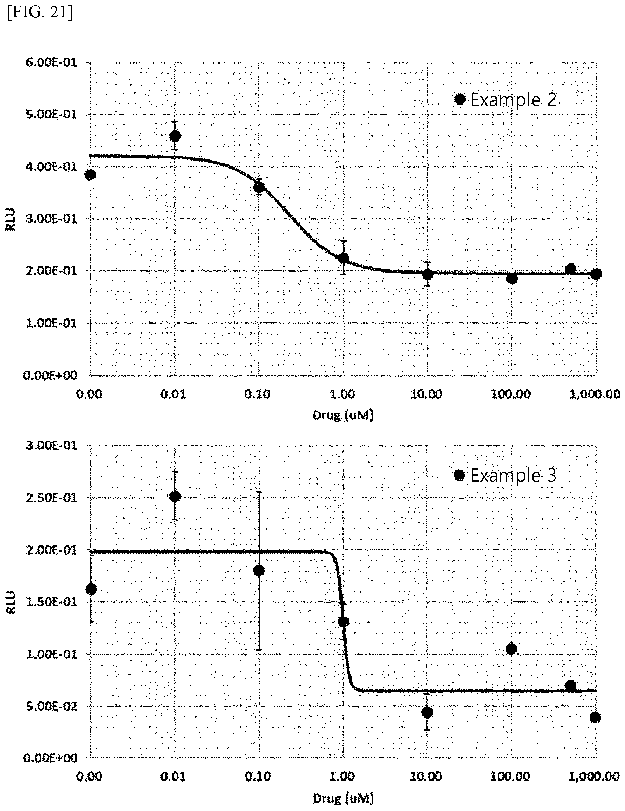

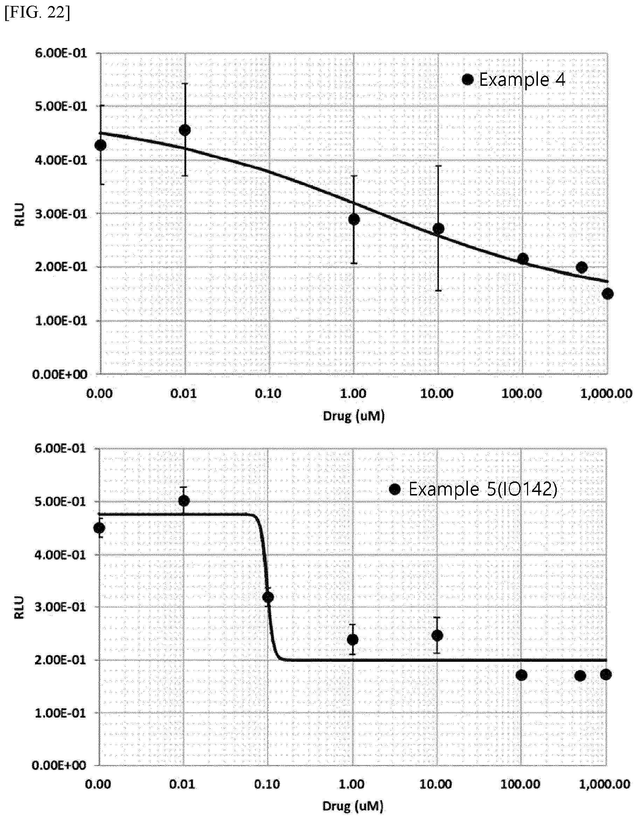

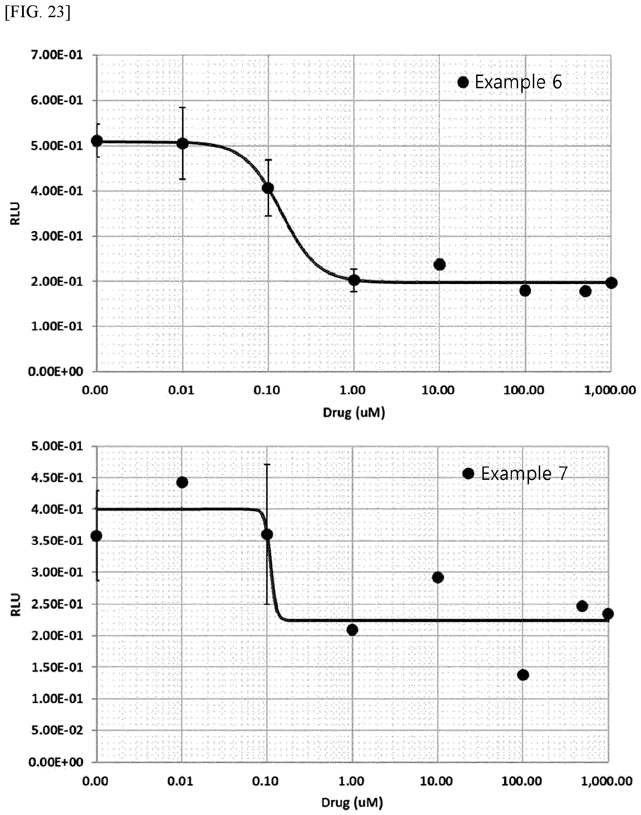

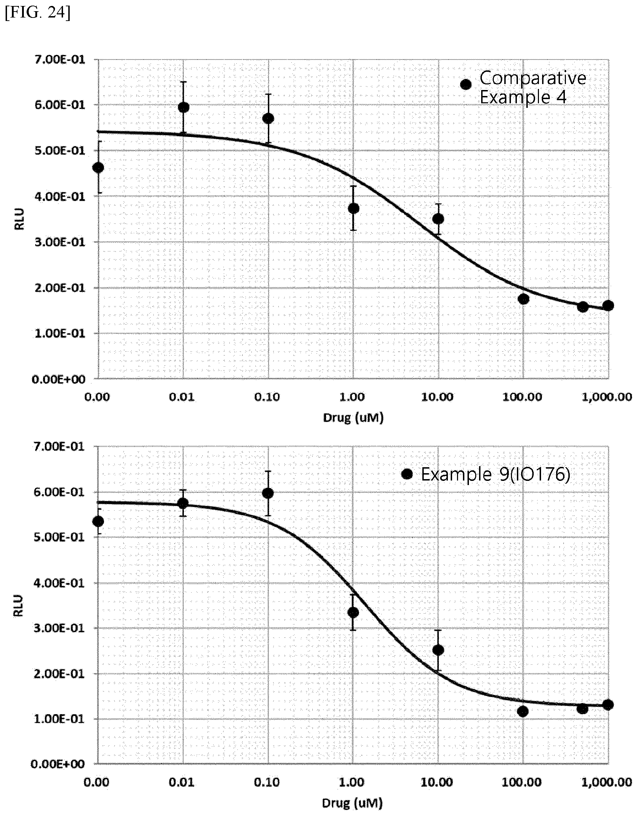

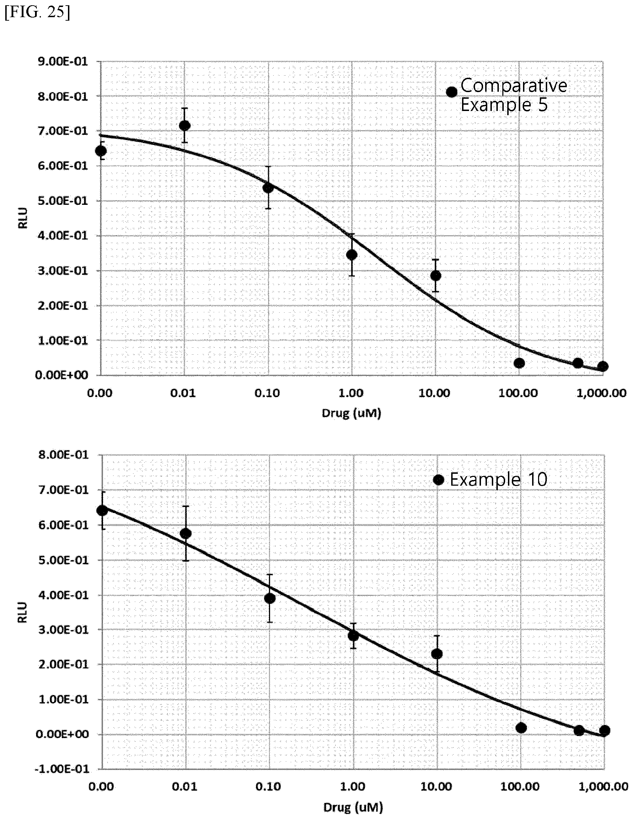

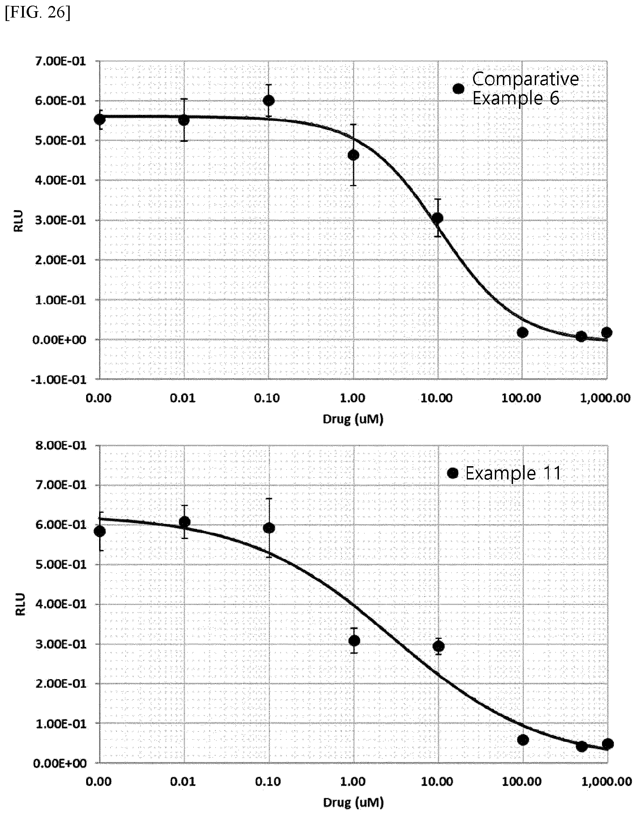

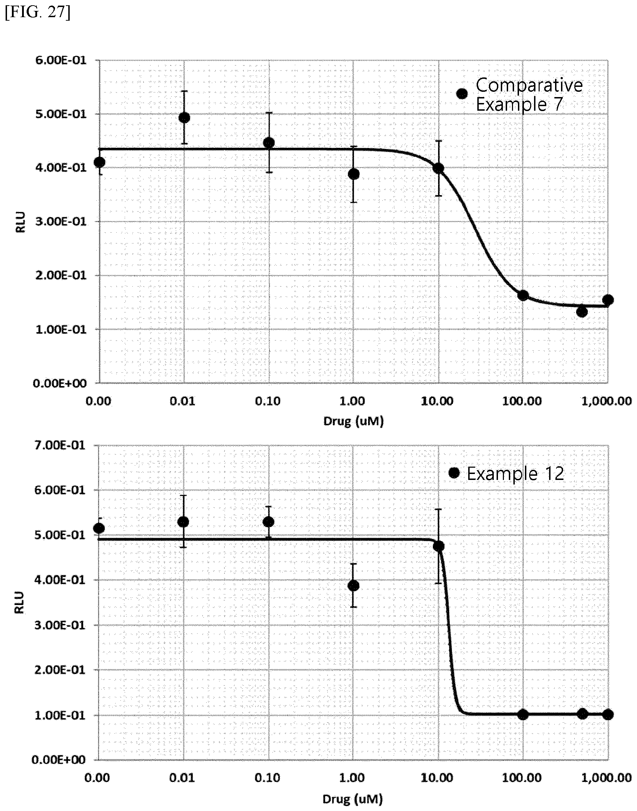

The cells cultured by the above-described method were dispensed by 2 x 10⁴ into each well of a 96-well culture vessel and cultured for 24 hours. The medium of the cells attached to the culture vessel was replaced with an ATCC modified RPMI-1640 medium containing 5% FBS, and samples of the above-described modified oligonucleotide-drug conjugates (Examples 1-7 and 9-12, and Comparative Example 1-7, respectively) were treated at concentrations of 0, 0.01, 0.1, 1, 10, 100, 500 and 1000 µM. After 72 hours, 10 µl of WST reagent was added to each well and reacted for 2 hours. The produced formazan was measured with an absorbance at 450 nm using a Glomax plate meter (Promega, USA), and IC₅₀ was evaluated according to the protocol in the device. IC₅₀ evaluation results and graphs illustrating the same are shown in Table 2 below and FIGS. 19 to 27 (in Table 2, M = Gemcitabine, and N = 5-FdU).

**[TABLE 2]**

| **Item** | **Configuration of examples (modified oligonucleotide-drug conjugates) and comparative examples** | | **IC₅₀** (µM) |
|---|---|---|---|
| | **Sequence of oligonucleotide or modified oligonucleotide (5'→3')** | **Type of conjugated drug/conjugated position** | |
| Comparat ive Example 1 | GGTGGTGGTGGTTGTGGTGGTGGTG G (SEQ ID NO: 1) | - | 89.9 |
| Comparat ive Example 2 | GGTGGTGGTGGTTGUGGTGGTGGTG G (SEQ ID NO: 2) | Paclitaxel/conjugated to U of SEQ ID NO: 2 (15th nucleotide of SEQ ID NO: 2) | 4.59 |
| Comparat ive Example 3 | GGTGGTGGTGGTUGTGGTGGTGGTG G (SEQ ID NO: 3) | Paclitaxel/conjugated to U of SEQ ID NO: 3 (13th nucleotide of SEQ ID NO: 3) | 4.04 |
| Example 1 | GGTGGTGGTGGUMGTGGTGGTGGTG G (SEQ ID NO: 4) | Paclitaxel/conjugated to U of SEQ ID NO: 4 (12th nucleotide of SEQ ID NO: 4) | 2.21 |
| Example 2 | GGTGGTGGTGGUTMTGGTGGTGGTG G (SEQ ID NO: 5) | Paclitaxel/conjugated to U of SEQ ID NO: 5 (12th nucleotide of SEQ ID NO: 5) | 0.23 |
| Example 3 | GGTGGTGGTGGTUMTGGTGGTGGTG G (SEQ ID NO: 6) | Paclitaxel/conjugated to U of SEQ ID NO: 6 (13th nucleotide of SEQ ID NO: 6) | 1.00 |
| Example 4 | GGTGGTGGTGGTUGMGGTGGTGGTG G (SEQ ID NO: 7) | Paclitaxel/conjugated to U of SEQ ID NO: 7 (13th nucleotide of SEQ ID NO: 7) | 1.61 |
| Example 5 (IO142) | GGTGGTGGTGGMUGMGGTGGTGGT GG (SEQ ID NO: 8) | Paclitaxel/conjugated to U of SEQ ID NO: 8 (13th nucleotide of SEQ ID NO: 8) | 0.1 |
| Example 6 | GGTGGTGGTGGTUGTGGTGGTGGMG G (SEQ ID NO: 9) | Paclitaxel/conjugated to U of SEQ ID NO: 9 (13th nucleotide of SEQ ID NO: 9) | 0.14 |
| Example 7 | GGTGGTGGTGGTTGUGGTGGTGGMG G (SEQ ID NO: 10) | Paclitaxel/conjugated to U of SEQ ID NO: 10 (15th nucleotide of SEQ ID NO: 10) | 0.11 |
| Example 8 | GGTGGTGGTGGTTGTGGTGGTGGTGG MM (SEQ ID NO: 11) | Paclitaxel/conjugated to 5'-end (i.e., 1st nucleotide) of SEQ ID NO: 11 | 5.97 |
| Comparat ive Example 4 | GGTGGTGGTGGTTGTGGTGGTGGTGG (SEQ ID NO: 1) | Monomethyl auristatin E (MMAE)/conjugated to 5'-end (i.e., 1st nucleotide) of SEQ ID NO: 1 | 1.39 |
| Example 9 (IO176) | GGTGGTGGTGGTTGTGGTGGTGGTGG MM (SEQ ID NO: 11) | MMAE/conjugated to 5'-end (i.e., 1st nucleotide) of SEQ ID NO: 11 | 1.97 |
| Comparat ive Example 5 | GGTGGTGGTGGTTGTGGTGGTGGTGG (SEQ ID NO: 1) | DM1/conjugated to 5'-end (i.e., 1st nucleotide) of SEQ ID NO: 1 | 0.26 |
| Example 10 | GGTGGTGGTGGTTGTGGTGGTGGTGG MM (SEQ ID NO: 11) | DM1/conjugated to 5'-end (i.e., 1st nucleotide) of SEQ ID NO: 11 | 10.44 |
| Comparat ive Example 6 | GGTGGTGGTGGTUGTGGTGGTGGTG G (SEQ ID NO: 3) | DM1/conjugated to U of SEQ ID NO: 3 (13th nucleotide of SEQ ID NO: 3) | 3.01 |
| Example 11 | GGTGGTGGTGGMUGMGGTGGTGGT GG (SEQ ID NO: 8) | DM1/conjugated to U of SEQ ID NO: 8 (13th nucleotide of SEQ ID NO: 8) | 26.71 |
| Comparat ive Example 7 | GGTGGTGGTGGTTGTGGTGGTGGTGG (SEQ ID NO: 1) | Paclitaxel/conjugated to 5' of SEQ ID NO: 1 | 13.21 |
| Example 12 | GGTGGTGGTGGTTGTGGTGGTGGTGG NN (SEQ ID NO: 12) | Paclitaxel/conjugated to 5' of SEQ ID NO: 12 | 13.21 |

The IC₅₀ of Comparative Example 1, which is a control aptamer, is 89.9 µM. Meanwhile, the modified oligonucleotide-drug conjugates of the examples exhibited IC₅₀ values which were several to dozens of times lower than those of Comparative Example 1, which is the control aptamer.

In addition, it was confirmed that the examples (e.g., Examples 1 to 12) in which drugs are conjugated to some amino acids in the sequence of Comparative Example 1, and other amino acids are replaced with modified nucleic acids, or further include the modified nucleic acids exhibited IC₅₀ values which were at least 2 times and at most 40 times lower than those of the comparative examples (Comparative Examples 2 to 7) in which only drugs are conjugated to some amino acids without replacement with the modified nucleic acids.

This means that the modified oligonucleotide-drug conjugate of the present invention may exhibit superior anticancer effects than the control aptamer.

### 3. Confirmation of anticancer efficacy of modified oligonucleotide-drug conjugates - animal experiment (in vivo)

Example 5 (IO142) and Example 9 (IO176) with excellent IC₅₀ measurement results were selected and animal experiments were performed.

### (1) Preparation of 'patch-type collagen drug delivery system' loading modified oligonucleotide-drug conjugate

To deliver the modified oligonucleotide-drug conjugates to an animal model, high-purity collagen (COLTRIX^{®}Tendoregen, Ubiosis Co., Ltd.) dispersion (1.0%) and samples (e.g., modified oligonucleotide-drug conjugates, gemcitabine, etc.) to be tested were mixed, poured into a circular silicone mold having a diameter of 1 cm, and lyophilized to prepare a "patch-type collagen drug delivery system" in which the samples were loaded in the collagen. Amounts of the samples loaded per patch-type collagen drug delivery system are as shown in Table 3 below (in Table 3, SEQ ID NO: 16 is 5'-(Gem)(Gem)[TGG]₄[TTG][TGG]₅-3', wherein Gem=Gemcitabine).

**[TABLE 3]**

| **Item** | **Sample loaded in collagen patch** | **Loading amount (mg/patch)** |
|---|---|---|
| Comparative Experimental Example 1 | Gemcitabine | 0.12 |
| Comparative Experimental Example 2 | IO101L (SEQ ID NO: 16) | 2.0 |
| Experimental Example 1 | Example 9 (Modified oligonucleotide-drug conjugate in which drug is conjugated to 5'-end of SEQ ID NO: 11,10176) | 0.2 |
| Experimental Example 2 | Example 5 (Modified oligonucleotide-drug conjugate in which drug is conjugated to U of SEQ ID NO: 8, IO142) | 2.0 |
| Comparative Experimental Example 3 | - (No sample added, collagen alone) | - |

Specifically, each of sample (gemcitabine, IO101L (SEQ ID NO: 16) and modified oligonucleotide-drug conjugates (Example 5 and Example 9) to be tested was added to the high-purity collagen, and then uniformly mixed using a multi-mixer (SLRM-3, MYLAB) at 4°C for 1 hour. Thereafter, a predetermined amount of the collagen-drug mixture was dispensed into a cylindrical silicone mold having a diameter of 1 cm and the collagen-drug mixture was primary frozen at -20 °C for 4 hours or more. After the first freezing was completed, the sample was separated from the cylindrical silicone mold and transferred to a sterile dish or plate, and then secondary frozen at - 80 °C for 2 hours or more. After preliminary freezing so that the cold trap temperature of a lyophilizer was -80°C, the mixture was placed in the lyophilizer and lyophilized for more than 16 hours. The lyophilized mixture was compressed in the form of a patch using an acrylic plate, then was put into an aluminum pouch and sealed. The packaged patch-type collagen drug delivery system was refrigerated at 4°C.

Hereinafter, the patch-type collagen drug delivery system prepared by the above-described method is expressed as a patch or drug-loading collagen patch.

### (2) Safety and effectiveness evaluation of 'patch-type collagen drug delivery system' loading modified oligonucleotide-drug conjugate in animal model

### 1) Construction of orthotopic pancreatic cancer animal model and implantation of patch-type collagen drug delivery system

The frozen cancer cell line (BxPC-3-Luc) (Asan Institute for Life Sciences) was thawed, and then cultured in an RPMI1640 medium (Biowest, Cat#. L0498-100) in which 10% fetal bovine serum (Biowest, Cat#. S1480) and 1% antibiotic (Gibco, Cat#. 15240062) were supplied at 37°C under 5% CO₂ condition. The thawed cells were cultured for one week to confirm that there were no problems with the shape, survival rate, and doubling time.

Cancer cells having a cell survival rate of 95% or more were prepared in order to transplant them into mice. The cells were harvested using trypsin, and prepared so as to transplant them into 5×10⁵ cells/25 µl/mouse by being turbid in RPMI 1640 media. 6-week old BALB/c-nude male mice stabilized for 1 week (purchased from Jabio, Korea) were subjected to abdominal anesthesia, and the pancreas was taken out by dissecting the abdominal region of each anesthetized mouse and cells of 5×10⁵ cells/25 µl were transplanted. After 2 weeks, tumor formation was confirmed through luciferase imaging. Then, as shown in Table 4 below, the mice were divided into groups by 5 mice. The patch-type collagen drug delivery system prepared in the above (1) was inserted (intra-abdominal cavity insertion using surgery) into the tumor site of each mouse in the respective groups. All animal experiments included in the present test were performed according to the Institutional Animal Care and Use Committee (IACUC) of the Asan Institute for Life Sciences [2022-14-058].

**TABLE 4**

| **Group** | **Administration material** | **Administration route** | **Administration frequency** |
|---|---|---|---|
| Group 1 | Untreated | - | - |
| Group 2 | Gemcitabine 0.12 mg-loading collagen patch | Patch insertion | Once |
| Group 3 | IO101L 2.0 mg-loading collagen patch | Patch insertion | Once |
| Group 4 | Example 9 (IO176) 0.2 mg-loading collagen patch | Patch insertion | Once |
| Group 5 | Example 5 (IO142) 2.0 mg-loading collagen patch | Patch insertion | Once |

### 2) Effectiveness evaluation using IVIS imaging

After inserting the patch-type collagen drug delivery system, changes in the tumor size were observed using IVIS imaging equipment (Xenogen IVIS spectrum system; Caliper Life Science, Inc., Hopkinton, MA) at an interval of 3 times a week for 4 weeks to check the body weight. Luciferin was injected intraperitoneally at a concentration of 150 mg/kg per mouse, allowed to react for 7 minutes, and then IVIS (in vivo imaging system) imaging was performed. On the 28th day after inserting the patch-type collagen drug delivery system, IVIS imaging was performed, and the mouse was sacrificed after the body weight measurement was completed. Then, major organs (heart, lung, spleen, kidney and liver), pancreas and tumor were extracted to measure organ weights, followed by taking images. Since the mice needed time to recover on the day of insertion of the patch-type collagen drug delivery system, IVIS (in vivo imaging system) imaging was performed the day after insertion.

### 2-1) IVIS whole body image

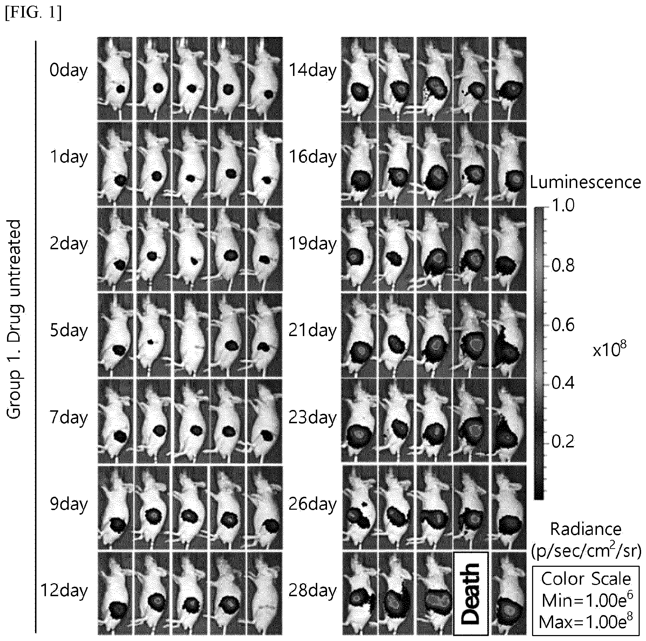

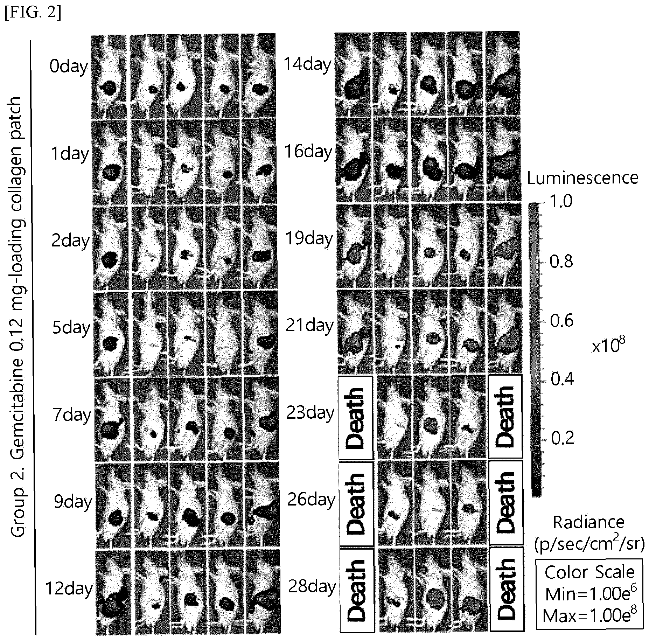

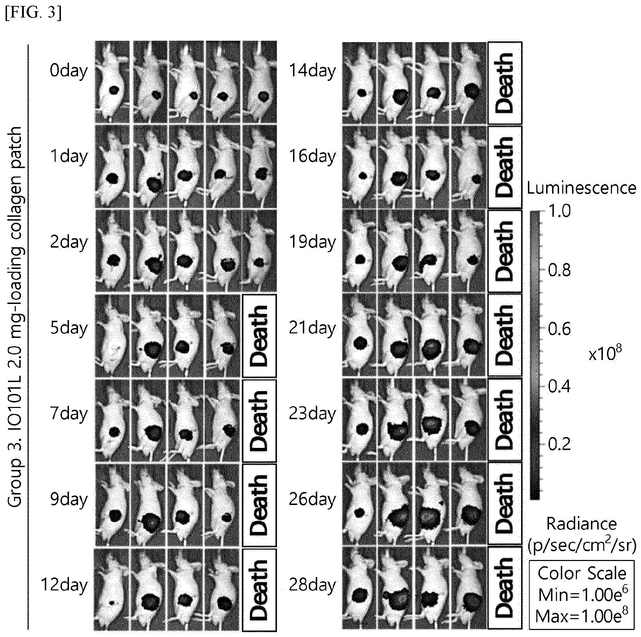

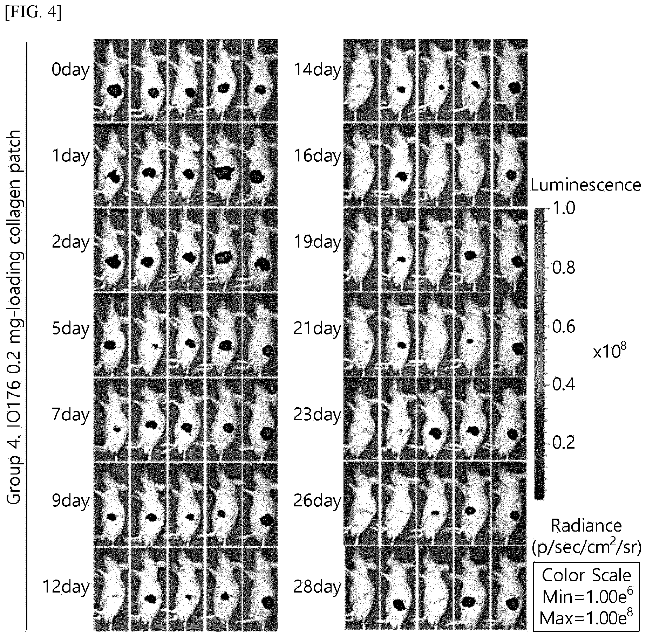

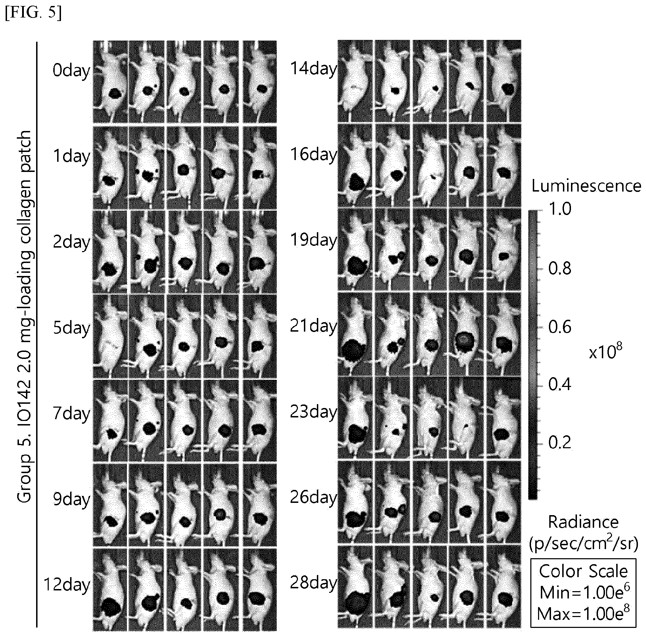

In the case of a drug-untreated mouse group (Group 1) and a group of mice inserted with IO101L 2.0 mg-loading collagen patch (Group 3), there were dead mice, and luminescence was gradually increased over time. In the case of a group of mice inserted with Example 9 (IO176) 0.2 mg-loading collagen patch (group 4), and a group of mice inserted with Example 5 (IO142) 2.0 mg-loading collagen patch (group 5), changes in the fluorescence were decreased. Here, an increase in the luminescence indicates the proliferation of cancer, and a decrease in the luminescence indicates that an effect of inhibiting tumor appears (see FIGS. 1 to 5).

### 2-2) Change in body weight

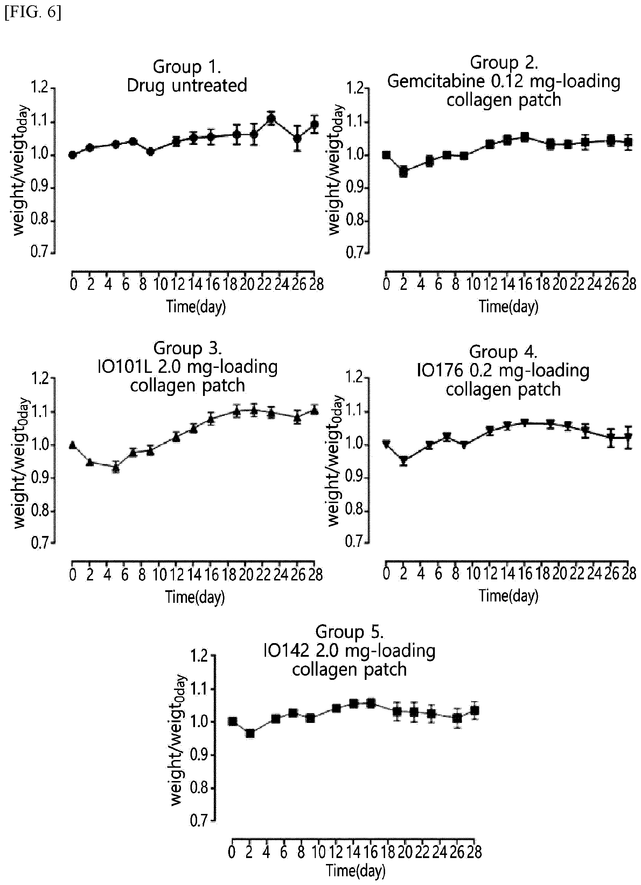

After inserting the patch-type collagen drug delivery system, the mouse body weight was measured at an interval of 3 times a week for 4 weeks. From the measurement results for each day, the measured body weights were divided by the body weight on day 0 and percentage changes in the mouse body weight compared to day 0 were observed. In all mouse groups (groups 2 to 5) inserted with the patch-type collagen drug delivery system, it was confirmed that the body weight was decreased for 2 to 6 days after the test substance was inserted, and then the body weight was gradually returned (see FIG. 6).

### 2-3) Imaging and weighing of extracted organs

On the 28th day after insertion of the patch-type collagen drug delivery system, the mice were sacrificed, and major organs (heart, lung, spleen, kidney and liver), pancreas and tumor were extracted and photographed, and organ weights were measured.

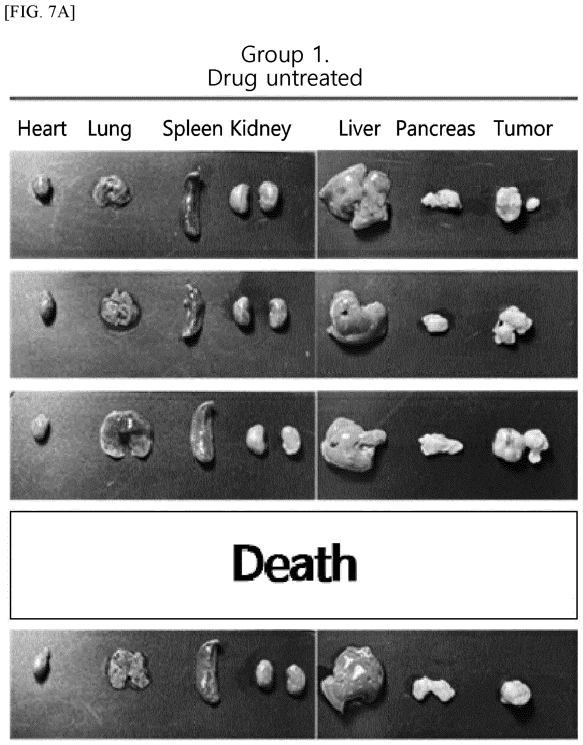

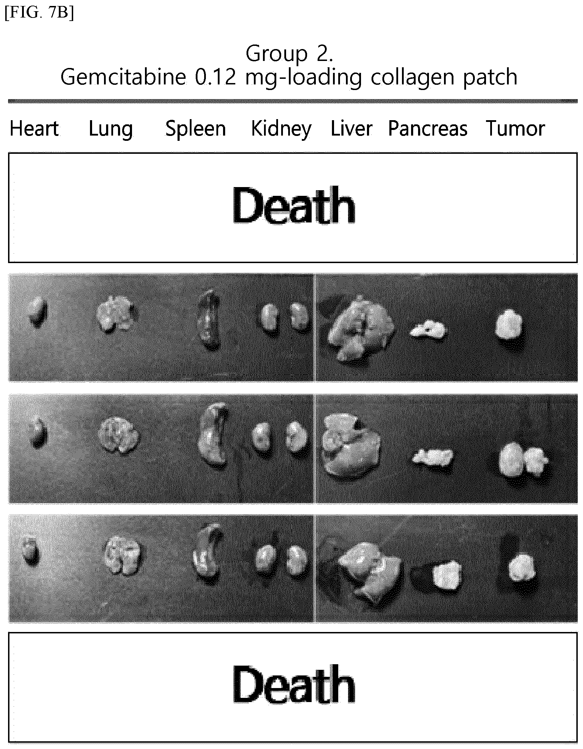

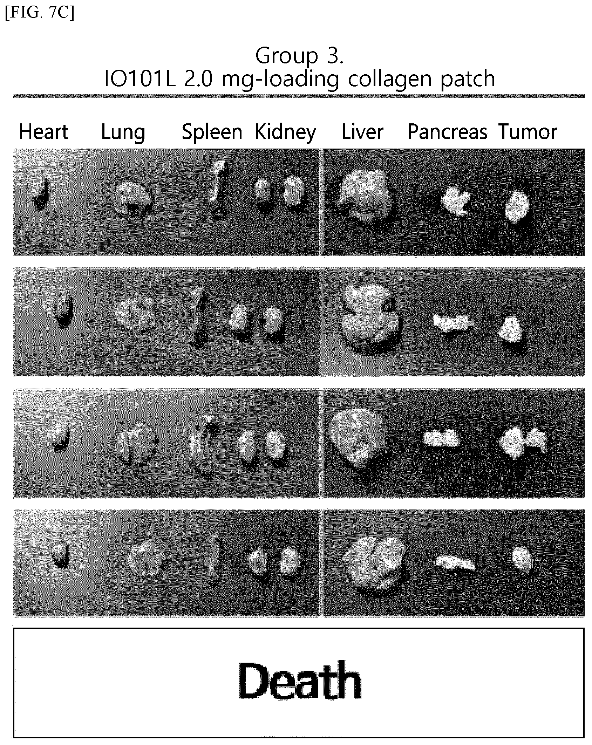

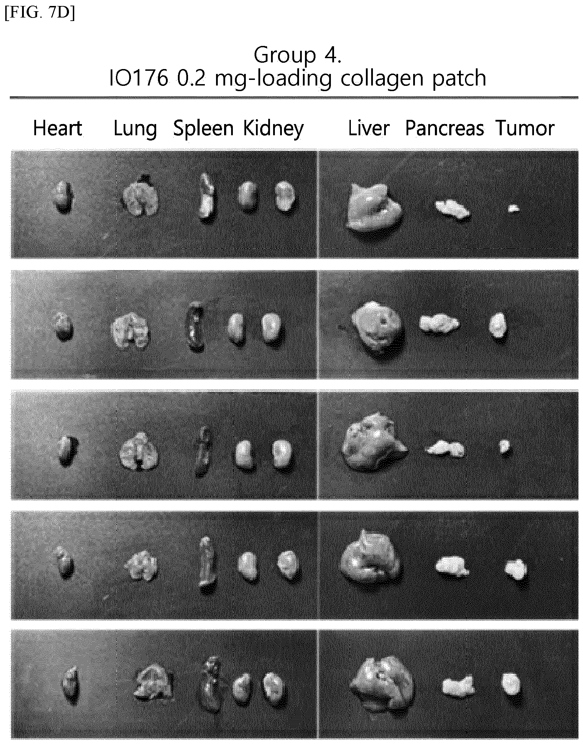

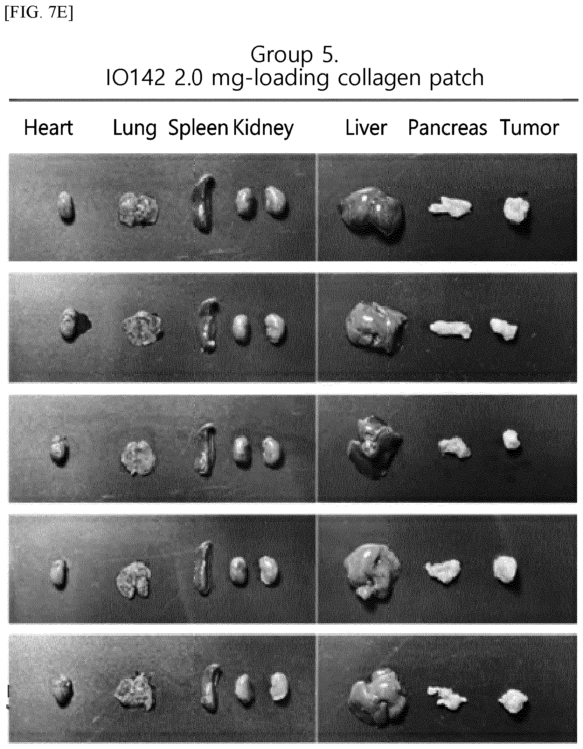

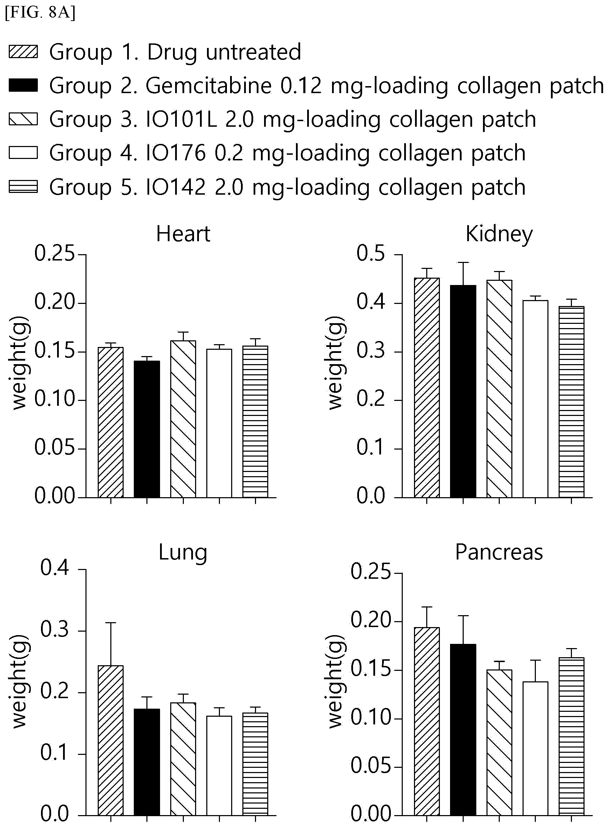

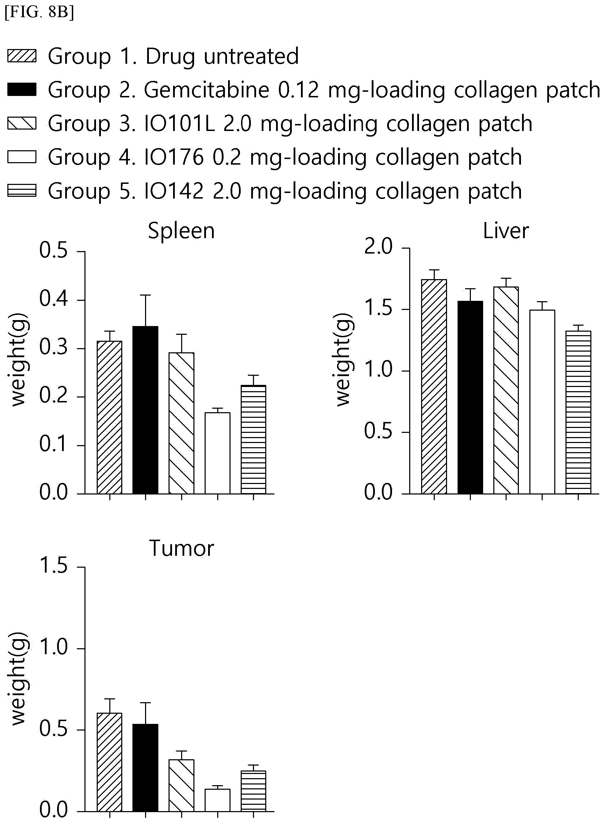

As shown in the experimental results of 2-2) above, the group of mice inserted with Example 9 (IO176) 0.2 mg-loading collagen patch (group 4) had the smallest tumor size, and no metastatic tumor was observed. In addition, the group of mice inserted with Example 9 (IO176) 0.2 mg-loading collagen patch (group 4) had the lowest tumor weight, and lower weights were observed in the order of the group of mice inserted with Example 5 (IO142) 2.0 mg-loading collagen patch (group 5), and the group of mice inserted with IO101L 2.0 mg-loading collagen patch (Group 3) (see FIGS. 7A to 7E, and FIG. 8).

From the IVIS whole body images, tumor size and weight measurement results, it was confirmed that, in the group of mice inserted with Example 9 (IO176) 0.2 mg-loading collagen patch (Group 4) and the group of mice inserted with Example 5 (IO142) 2.0 mg-loading collagen patch (Group 5), no dead mouse was observed and excellent effect of inhibiting tumor appeared. In addition, as a result of dissecting the mice in the test groups, no administered drug-loading collagen patch was observed, which suggests that all the patches were dissolved and absorbed in the mice.

### 4. Pharmacokinetic evaluation of modified oligonucleotide-drug conjugates - using rats

After 40 mg/kg of Example 9 (IO176), 4 mg/kg of MMAE (MedChemExpress, Cat#. HY-15162) of the same amount as the Monomethyl auristatin E (hereinafter referred to as MMAE) included in Example 9, and 2 mg/kg of gemcitabine (BetaPharma (Shanghai) Co., Ltd, Cat#. 86-39157) of the same amount as the gemcitabine included in Example 9 were intravenously administered into rats through the jugular vein, respectively, i) pharmacokinetic coefficients of MMAE and ii) pharmacokinetic coefficients of gemcitabine and dFdU, an inactive metabolite of the gemcitabine, were calculated and compared with each other.

Specifically, Sprague-Dawley male rats (8 weeks old, purchased from Youngbio) were acclimatized for 1 week in the animal room of the Catholic University Songsim Campus, and then pharmacokinetic experiments were performed. Rats were induced with isoflurane, an inhalation anesthetic, and PE50 (polyethylene tubing, Clay Adams, Becton Dickinson, NJ, USA) was inserted into the carotid (for blood collection) and jugular vein (for drug administration), followed by suturing with suture, and then end portions thereof were fixed to the back of the neck. During the surgery, anesthesia was maintained using ether, and about 0.5 mL of physiological saline solution containing heparin (20 units/mL) was injected to prevent blood from coagulating in the cannula. After completing the surgery, each rat was placed in a metabolic cage and allowed to fully recover from the anesthetic (for 4-5 hours). Then, the rats were divided into three groups of a group administered with Example 9 (IO176) alone (n=6), and a group administered with MMAE alone (n=7), and a group administered with gemcitabine alone (n=6), and each sample (Example 9, MMAE alone, gemcitabine alone) was administered into the rats. All of Example 9, MMAE and gemcitabine were dissolved in physiological saline at 2 mL per kg of body weight and administered into the jugular vein at doses of 40 mg/kg, 4 mg/kg and 2 mg/kg, respectively. When collecting blood, in order to prevent gemcitabine in 0.2 mL of blood from being converted into metabolites by cytidine deaminase, a light-shielding Eppendorf tube was prepared in which 2 µL of a solution containing tetrahydrouridine (hereinafter referred to as THU), a cytidine deaminase inhibitor, dissolved in distilled water at a concentration of 10 mg/mL was added in advance. 0.2 mL of blood was collected in each light-shielding Eppendorf tube that had been stored frozen in advance through the carotid in three groups immediately before each drug administration (0 min), 1, 5, 10, 15 and 30 minutes, and 1, 2, 4, 6, 8, 10, 20, 24 and 30 hours. Immediately after completing the blood collection, the plasma that had been stored in an ice-bath was quickly centrifuged at 4°C, and 100 µL of the plasma was dispensed into the light-shielding Eppendorf tube. The dispensed plasma was quickly stored in an ice-bath and maintained at -80°C until LC-MS/MS analysis was performed. After collection of blood from the carotid, the rats were euthanized in a CO₂ gas chamber.

### (1) Plasma concentration analysis method

### 1) Concentration analysis method of MMAE

After pretreating samples by protein precipitation, plasma concentration of MMAE was analyzed using liquid chromatography-tandem mass spectrometry (hereinafter referred to as LC-MS/MS) established in the Pharmacology/Pharmacokinetics Laboratory of the Catholic University College of Pharmacy. All analysis processes were conducted under light-shielding conditions.

### ① LC-MS/MS conditions

- Analsis equipment: Agilent 1290 series HPLC, Qtrap 5500 LC-MS/MS
- Detector: Tandem mass spectrometer (triple quadrouple type)
   Ion source: Electrospray ionization (positive ion mode)
   Desolvation temperature: 500°C
   Nebulizing gas: Nitrogen, Collision gas: Nitrogen
   Quantitation: MRM (multiple reaction monitoring) mode
- Stationary phase: Luna C18 (2.0 × 100 mm, 3 µm, Phenomenex, California, USA)
- Stationary phase temperature: 40°C
- Mobile phase: Distilled water containing 0.1% formic acid (A)/acetonitrile containing 0.1% formic acid (B) (50:50, v/v) (see **[Table 5]** below)
- Flow rate: 0.3 mL/min
- Mass parameters: See **[Table 6]** below (CE: Collision energy)

**[TABLE 5]**

| **Time (min)** | **Flow rate (mL/min)** | **A (%)** | **B (%)** |
|---|---|---|---|
| 0.00 | 0.3 | 50 | 50 |
| 2.00 | 0.3 | 50 | 50 |

**[TABLE 6]**

| **Analyte** | **Parent ion (*m*/*z*)** | **Product ion (*m*/*z*)** | **Collision energy (eV)** | **Retention time (Min)** |
|---|---|---|---|---|
| **MMAE 1 (for quantitative)** | 718.456 | 685.5 | 39 | 0.712 |
| **MMAE 2 (for qualitative)** | 718.456 | 152.1 | 39 | 0.712 |
| **Verapamil (IS)** | 455.248 | 165.1 | 35 | 0.725 |

### ② Construction of calibration curve

MMME standard products were dissolved in acetonitrile to prepare a 1 mg/mL of storage solution and store it frozen. Then, the storage solution was diluted with acetonitrile to prepare working solutions so that the concentrations were 0.250, 0.500, 2.50, 5.00, 12.5 and 50.0 µg/mL, respectively, followed by storing them in a refrigerator. Verapamil, an internal standard (IS), was dissolved in acetonitrile to prepare 200 ng/mL of working solution. The working solution of MMAE was added to rat blank plasma containing THU to prepare standard plasma samples so that the plasma concentrations of MMAE were 10, 20, 100, 200, 500 and 2000 ng/mL, respectively.

### ③ Plasma sample treatment method

200 µL of verapamil (200 ng/mL), the internal standard dissolved in acetonitrile, was added to 25 µL of rat plasma, and vortexed for 10 minutes, then centrifuged at 14,000 rpm at 4°C for 5 minutes. Thereafter, 190 µL of the supernatant was taken and transferred to an LC-MS/MS vial through a 0.2 µm syringe filter, and then 2 µL thereof was injected and analyzed by LC-MS/MS.

### ④ Determination of suitability of analysis

In order to determine the suitability of the analysis process, samples for constructing a calibration curve were analyzed for each batch of analysis samples during sample treatment, then samples for suitability of analysis (lowest limit of quantification for MMAE: LLoQ, 10 ng/mL, low concentration: LoQC, 25 ng/mL, medium concentration: MiQC, 250 ng/mL, high concentration: HiQC, 1500 ng/mL) were analyzed twice. It was confirmed whether at least 67% of 6 (e.g., 4 out of 6) samples for suitability was within ±15% of the theoretical value, and 50% or more thereof was within ±15% of the theoretical value at the same concentration.

### ⑤ Calculation of plasma drug concentration

From the acquired chromatogram, a ratio of the peak area of MMAE to the peak area of the internal standard was obtained, and the plasma concentration of MMAE was calculated from the calibration curve constructed in advance.

### 2) Concentration analysis method of gemcitabine and dFdU

### ① LC-MS/MS conditions

- Analysis equipment: Agilent 1290 series HPLC, Qtrap 5500 LC-MS/MS
- Detector: Tandem mass spectrometer (triple quadrouple type)
   Ion source: Electrospray ionization (positive ion mode)
   Desolvation temperature: 500°C
   Nebulizing gas: Nitrogen, Collision gas: Nitrogen
   Quantitation: MRM (multiple reaction monitoring) mode
- Stationary phase: Hypersil gold C18 column (2.1 × 100 mm, 1.9 µm, Thermo)
   Fisher Scientific Inc., Boston, USA)
- Stationary phase temperature: 40°C
- Mobile phase: Distilled water (A) and
   acetonitrile (B) respectively containing 0.1% formic acid (95:5, v/v) (see [Table 7] below)
- Flow rate: 0.22 mL/min
- Mass parameters: See **[Table 8]** below (CE: Collision energy)

**[TABLE 7]**

| **Time (min)** | **Flow rate (mL/min)** | **A (%)** | **B (%)** |
|---|---|---|---|
| 0.00 | 0.22 | 95 | 5 |
| 5.00 | 0.22 | 95 | 5 |

**[TABLE 8]**

| **Analyte** | **Parent ion (*m*/*z*)** | **Product ion (*m*/*z*)** | **Collision energy (eV)** | **Retention time (Min)** |
|---|---|---|---|---|
| **Gemcitabine** | 264.311 | 112.0 | 15 | 1.90 |
| **dFdU** | 265.000 | 113.0 | 15 | 3.52 |
| **5'-Deoxy-5-fluorocytidine (IS)** | 246.100 | 130.4 | 20 | 3.15 |

### ② Construction of calibration curve

Each of gemcitabine and dFdU standard product was dissolved in distilled water to prepare 400 µg/mL and 700 µg/mL of storage solutions and store them frozen. Then, the storage solutions were diluted with tertiary distilled water to prepare working solutions so that the concentrations were 0.200, 0.400, 0.800, 2.00, 10.0 and 40.0 µg/mL, respectively, followed by storing them in a refrigerator. 5'-Deoxy-5-fluorocytidine, an internal standard (IS), was dissolved in acetonitrile to prepare 50 ng/mL of working solution. The working solution of gemcitabine and dFdU was added to rat blank plasma containing THU to prepare standard plasma samples so that the plasma concentrations of gemcitabine and dFdU were 10, 20, 40, 100, 500 and 2000 ng/mL, respectively.

### ③ Plasma sample treatment method

150 µL of 5'-Deoxy-5-fluorocytidine (50 ng/mL), the internal standard dissolved in acetonitrile, was added to 20 µL of rat plasma, and vortexed for 10 minutes, followed by centrifuging at 14,000 rpm at 4°C for 5 minutes. Thereafter, 150 µL of the supernatant was taken and transferred to another Eppendorf tube, and then the organic solvent was dried under a nitrogen stream. 100 µL of tertiary distilled water was added to the dried Eppendorf tube to be redissolved. Then, after vortexing for 10 minutes and centrifuging at 14,000 rpm at -4°C for 5 minutes, 90 µL of the supernatant was taken. Thereafter, 90 µL of the supernatant was taken and transferred to another Eppendorf tube, followed by centrifuging at 14,000 rpm at -4°C for 5 minutes. 80 µL of the supernatant was taken and transferred to an LC-MS/MS vial, and then 5 µL thereof was injected and analyzed by LC-MS/MS.

### ④ Determination of suitability of analysis

In order to determine the suitability of the analysis process, samples for constructing a calibration curve were analyzed for each batch of analysis samples during sample treatment, then samples for suitability of analysis (respective lowest limits of quantitation for gemcitabine and dFdU: LLoQ, 10 ng/mL; low concentration: LoQC, 30 ng/mL, medium concentration: MiQC, 300 ng/mL, high concentration: HiQC, 900 ng/mL) were analyzed twice. It was confirmed whether at least 67% of 6 (e.g., 4 out of 6) samples for suitability was within ±15% of the theoretical value, and 50% or more thereof was within ±15% of the theoretical value at the same concentration.

### ⑤ Calculation of plasma drug concentration

From the acquired chromatogram, a ratio of the peak areas of gemcitabine and dFdU to the peak area of the internal standard was obtained, and the plasma concentrations of gemcitabine and dFdU were calculated from the calibration curve constructed in advance.

### 3) Pharmacokinetic parameter calculation method and statistical processing

Pharmacokinetic parameters of MMAE, gemcitabine and dFdU were analyzed by non-compartment analysis using the Phoenix Winnonlin^{™} (6.4 version, Certara) program. Area under the plasma concentration-time curve (AUCt) values were obtained using the log-linear trapezoidal rule from plasma concentration-time curves to the final quantification time after drug administration. Area under the plasma concentration-time curve extrapolated to infinity (AUCinf) values were obtained using Equation 1 below. The terminal elimination rate constant (γZ) and half-life (t1/2) were obtained from the slope of the elimination phase of the plasma concentration trend. AUCinf = AUCt + Ct/γZ (Ct:final quantitative concentration, γZ: terminal elimination rate constant)

Statistical processing for comparison of pharmacokinetic parameters of each two groups was performed using t-test.

### (2) Plasma concentration analysis results

### 1) Concentration analysis results of MMAE

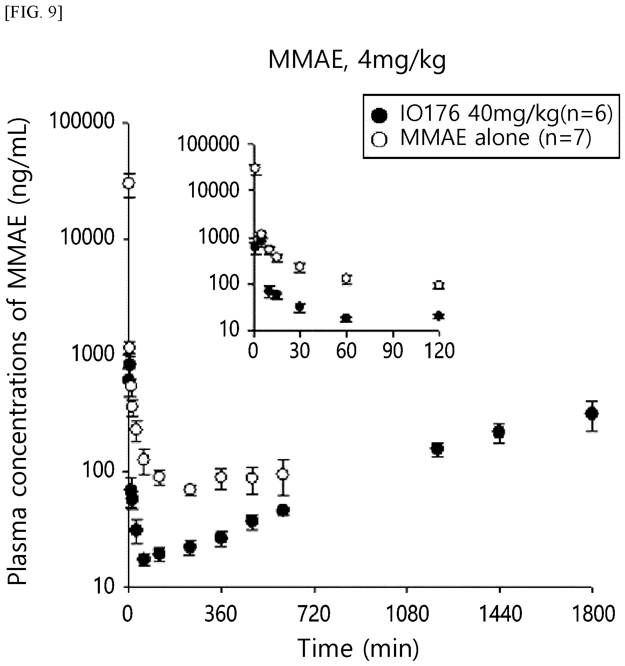

When intravenously administering 40 mg/kg of Example 9 (IO176) and intravenously administering 4 mg/kg of MMAE alone of the same amount to the rats, i) the plasma concentrations of MMAE are shown in FIG. 9, and ii) the pharmacokinetic coefficients of MMAE are summarized in **[Table 9]** below.

When intravenously administering 4 mg/kg of MMAE, all tested 7 rats were died 480 to 600 minutes after administration. However, in Example 9 (IO176) 40 mg/kg intravenous administration group, the plasma concentration of MMAE at 1 minute after administration was 605 ± 165 ng/mL, which was detected to be lower about 50 times than the MMAE 4 mg/kg alone intravenous administration group (29640 ± 7190 ng/mL), the plasma concentration of MMAE after administration was slowly increased from 60 to 120 minutes until 1800 minutes, the last time of blood collection, and all the tested rats were alive without dying (see FIG. 9). The AUC value of MMAE up to 120 minutes, the initial time after administration, was 71.7 ± 9.61 µg min/mL for the MMAE 4 mg/kg alone intravenous administration group, which was calculated to be higher about 9.70 times than the Example 9 (IO176) 40 mg/kg intravenous administration group (7.39±1.05 µg min/mL). However, the AUC value up to 1800 minutes, the last time of blood collection, was rather shown to be higher about 1.55 times in the Example 9 (IO176) 40 mg/kg intravenous administration group (see Table 9 below).

It is expected that, because the rate at which MMAE conjugated to Example 9 (IO176) is released into the plasma was very slow, Example 9 might show a statistically significantly lower plasma concentration of MMAE, and thereby the cytotoxicity of MMAE would be reduced, such that no death individual would have appeared in the tested rats.

**[TABLE 9]**

| PK parameters | M-A-G (n=6) | MMAE (n=7) | P |
|---|---|---|---|
| AUCₜ (µg min/mL) | 198±51.4 | 128±16.1 | 0.0056 |
| AUC₀₋₁₂₀ (µg min/mL) | 7.39±1.05 | 71.7±9.61 | 0.0000000005 |

### 2) Concentration analysis results of gemcitabine and dFdU

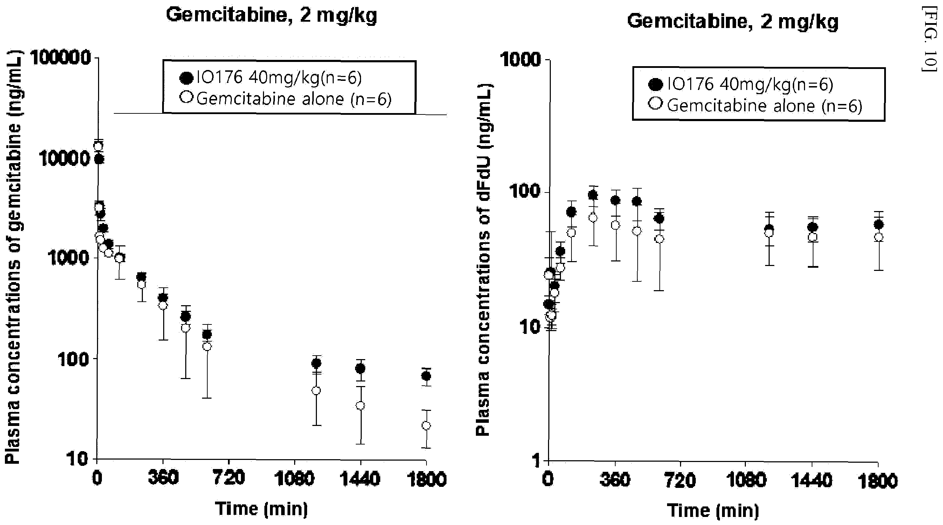

When intravenously administering 40 mg/kg of Example 9 (IO176) and intravenously administering 2 mg/kg of gemcitabine alone of the same amount to the rats, i) the plasma concentrations of gemcitabine and metabolite dFdU thereof are shown in FIG. 10, and ii) the pharmacokinetic coefficients of gemcitabine and metabolite dFdU thereof are summarized in [Table 10] below.

The analysis results for gemcitabine are as follows (see Table 10 below). The Example 9 (IO176) administration group exhibited a statistically significantly higher plasma concentration of gemcitabine than the gemcitabine alone intravenous administration group of the same amount [see the figure on left in FIG. 10]. As a result, the AUCt values of gemcitabine were 610±52.2 µg min/mL and 438±124 µg min/mL, respectively, which were calculated to be higher about 1.39 times. In addition, it was confirmed that the plasma half-life (t1/2) of gemcitabine was statistically significantly increased in the Example 9 (IO176) administration group. Through this, it was predicted that, since the initial release rate of gemcitabine in the IO176 (Example 9) administration group was high, the plasma concentration of gemcitabine would remain high, but the plasma half-life would be increased due to continuous release.

The analysis results for dFdU, an inactive metabolite of gemcitabine, are as follows (see Table 10 below). The plasma concentration tended to be slightly higher in the Example 9 (IO176) administration group, but the AUCt values were 111 ± 22.3 µg min/mL and 79.8 ± 44.2 µg min/mL, respectively, thereby showing no statistical significance. In the case of maximum plasma concentration (Cmax) of dFdU, the Example 9 (IO176) administration group exhibited a statistically significantly higher value. However, the metabolic conversion rates of inactive metabolites (AUCt, dFdU/AUCt, gemcitabine) in the two groups were 0.182±0.0263 and 0.170±0.0621, respectively, thereby showing no statistical significance. Through this, it can be seen that gemcitabine liberated into plasma from Example 9 (IO176) does not affect the conversion rate of inactive metabolite.

In Table 10 below, AUCt is an area under the curve from 0 minutes to the last blood collection time, t1/2 is a terminal half-life (plasma elimination half-life), CL is a total body clearance, Vdss is a body distribution volume, MRT is a mean residence time at which the drug stays in the body, Tmax is a median (ranges), and the metabolic conversion ratio value is a value calculated by dividing the dFdU AUCt value by the gemcitabine AUCt value.

**[TABLE 10]**

| **PK parameters** | | **M-A-G (n=6)** | **Gemcitabine (n=6)** | **P** |
|---|---|---|---|---|
| Gemcitabine | | | | |
| | AUCₜ (µg min/mL) | 610 + 52.2 | 438±124 | 0.0102 |
| | AUC_{inf} (µg min/mL) | 716±118 | 450±128 | 0.0038 |
| | t_{1/2}(min) | 984±543 | 408±167 | 0.0322 |
| | CL (mL/min/kg) | 2.35±0.440 | 4.77±1.43 | 0.0104 |
| | Vd₃₃ (mL/kg) | 3780±1610 | 2730±1220 | 0.2340 |
| | MRT (min) | 767±355 | 342±104 | 0.0183 |
| dFdU | | | | |
| | AUCₜ (µg min/mL) | 111±22.3 | 79.8±44.2 | 0.1486 |
| | Cₘₐₓ (ng/mL) | 99.3±18.5 | 68.8±26.2 | 0.0418 |
| | Tₘₐₓ (min)^{a} | 240 (240∼480) | 240 (240∼1200) | 0.5584 |
| Metabolic ratio^{b} | | 0.182±0.0263 | 0.170±0.0621 | 0.6714 |
| ^{a} Median (ranges). ^{b} AUCₜ, _{dFdU}/ AUC_{t. gemcitabine} | | | | |

[National research and development project that supported this invention]
[Project identification No.] 1425162626
[ProjectNo.] S3004867
[Ministry name] Ministry of SMEs and Startups
[Project management (professional) organization name] Technology and information Promotion Agency for SMEs
[Research project name] Technology Innovation Development of SMEs
[Research project name] New anticancer drug development technology using multidrug binding aptamer
[Contribution rate] 1/1
[Project progress organization name] Interoligo Co., Ltd.
[Research period] 20200921 - 20220920

## Claims

1. A modified oligonucleotide-drug conjugate comprising: a modified oligonucleotide represented by Formula 1 below; and a drug conjugated to deoxyuridine (dU) included in the modified oligonucleotide;
[Formula 1] 5'-(M₁)ₐ-(N₁)_{b}-GGX₁GGX₂GGX₃GGX₄X₅Y₁X₆GGX₇GGX₈GGX₉GG-(N₂)_{c}-(M₂)_{d}-3'
(in Formula 1 above,
X₁ to X₃ and X₇ to X₉ are each independently thymidine (T), or a modified nucleic acid represented by Formula 3 or Formula 4 below;
Yi is deoxyguanosine (dG), or a modified nucleic acid represented by Formula 3 or Formula 4 below;
X₄ to X₆ are each independently thymidine (T), deoxyuridine (dU), or a modified nucleic acid represented by Formula 3 or Formula 4 below, and at least one of X₄ to X₆ is deoxyuridine (dU);
M₁ and M₂ are each independently a modified nucleic acid represented by Formula 3 or Formula 4 below;
N₁ and N₂ are each independently thymidine (T), deoxyuridine (dU), deoxycytidine (dC), or deoxyguanosine (dG);
a and d are each independently an integer of 0 to 10, but when all of X₁ to X₉ are thymidine (T) and Yi is deoxyguanosine (dG), a and d are not 0 at the same time; and
b and c are each independently an integer of 0 to 10), 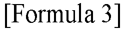 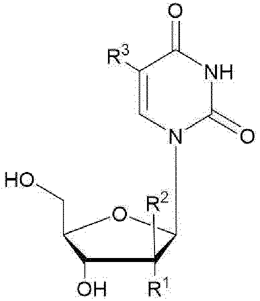 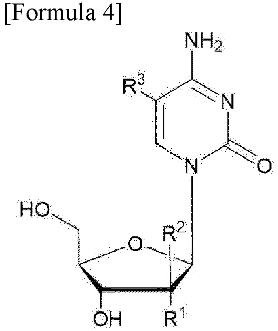
(in Formula 3 or Formula 4 above,
R¹ is hydrogen, halogen or a hydroxy group;
R² is hydrogen, halogen or a hydroxy group; and
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

2. The modified oligonucleotide-drug conjugate according to claim 1, wherein the modified nucleic acid represented by Formula 3 or Formula 4 above is selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluoro-2'-deoxycytidine and bromovinyldeoxyuridine.

3. The modified oligonucleotide-drug conjugate according to claim 1, wherein the drug is selected from the group consisting of paclitaxel, monomethyl auristatin E, monomethyl auristatin F, monomethyl auristatin D (MMAD), cytarabine, gemcitabine, maitansine, mertansine (DM1), DM4, calicheamicin and derivatives thereof, doxorubicin, duocarmycin and derivatives thereof, pyrrolobenzodiazepine (PBD), SN-38, α-amantine, tubulysin analog, cyclophosphamide, mecholrethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, auristatin E, auristatin F and nemorubicin.

4. The modified oligonucleotide-drug conjugate according to claim 1, wherein the drug and the deoxyuridine (dU) included in the modified oligonucleotide are conjugated by a linker L in which L₁ and L₂ are bound;
the L₁ is selected from the group consisting of acrylamide-C2-NH₂, C12-NH₂, C3-NH₂, acrylamide-C6-propanamide-SH, acrylamide-C6-NH₂, C6-NH₂, and C6-SH, which are selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier C2 dT, amino-modifier C6 dT, S-Bz-thiol-modifier C6-dT, phosphodiester bond and nucleotide, and then obtained through a deprotection process; and
the L₂ is selected from the group consisting of maleimidocaproyl-valine-citrulline-p-aminobenzoyloxycarbonyl (MC-Val-Cit-PAB), succinimidyl 4-(*N-*maleimidomethyl) cyclohexane-1-carboxylate (SMCC), succinic acid, hydrazone, peptide, disulfide, thioether, valine-citrulline, *N*-maleimidomethylcyclohexane-1-carboxylate (MCC), maleimidocaproyl, mercaptoacetamidocaproyl, *N*-succinimidyl 4-(2-pyridyldithio) pentanoate (SPP), *N*-succinimidyl 4-(2-pyridylthio) pentanoate (SPDB), phosphodiester bond and nucleotide.

5. The modified oligonucleotide-drug conjugate according to claim 1, wherein the modified oligonucleotide consists of sequences selected from SEQ ID NO: 4 to SEQ ID NO: 10.

6. A pharmaceutical composition for preventing or treating cancer comprising the modified oligonucleotide-drug conjugate according to any one of claims 1 to 5.

7. The pharmaceutical composition for preventing or treating cancer according to claim 6, wherein the cancer is selected from the group consisting of leukemia, lymphoma, breast cancer, liver cancer, gastric cancer, ovarian cancer, cervical carcinoma, glioma cancer, colon cancer, lung cancer, pancreatic cancer, prostate cancer, hepatoma, gastric adenocarcinoma, uterine cancer, bladder cancer, thyroid cancer, ovarian cancer, melanoma and cervical cancer.

8. A modified oligonucleotide-drug conjugate comprising: a modified oligonucleotide represented by Formula 2 below; and a drug conjugated to at least one of 5' and 3' ends of the modified oligonucleotide;
[Formula 2] 5'-(M₁)ₐ-(N₁)_{b}-GGX₁GGX₂GGX₃GGX₄X₅GX₆GGX₇GGX₈GGX₉GG-(N₂)_{c}-(M₂)_{d}-3'
(in Formula 2 above,
X₁ to X₉ are each independently thymidine (T), or a modified nucleic acid represented by Formula 3 or Formula 4 below;
M₁ and M₂ are each independently a modified nucleic acid represented by Formula 3 or Formula 4 below;
N₁ and N₂ are each independently thymidine (T), deoxyuridine (dU), deoxycytidine (dC), or deoxyguanosine (dG);
a and d are each independently an integer of 0 to 10, but when all of X₁ to X₉ are thymidine (T), a and d are not 0 at the same time; and
b and c are each independently an integer of 0 to 10), 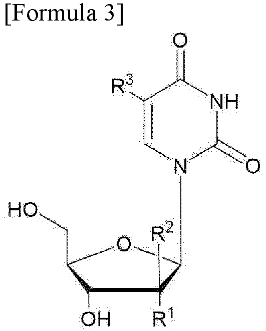 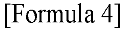 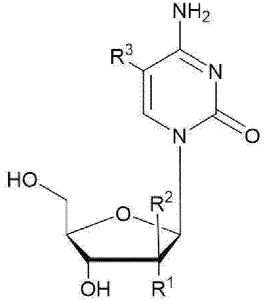
(in Formula 3 or Formula 4 above,
R¹ is hydrogen, halogen or a hydroxy group;
R² is hydrogen, halogen or a hydroxy group; and
R³ is hydrogen, halogen, a C1-C6 alkyl group, a C1-C6 haloalkyl group, a C2-C6 alkenyl group or C2-C6 haloalkenyl group (here, excluding the case where R¹ is hydrogen or a hydroxy group, R² is hydrogen and R³ is hydrogen or methyl)).

9. The modified oligonucleotide-drug conjugate according to claim 8, wherein the modified nucleic acid represented by Formula 3 or Formula 4 above is selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluoro-2'-deoxycytidine and bromovinyldeoxyuridine.

10. The modified oligonucleotide-drug conjugate according to claim 8, wherein the drug is selected from the group consisting of paclitaxel, monomethyl auristatin E, monomethyl auristatin F, monomethyl auristatin D (MMAD), cytarabine, gemcitabine, maitansine, mertansine (DM1), DM4, calicheamicin and derivatives thereof, doxorubicin, duocarmycin and derivatives thereof, pyrrolobenzodiazepine (PBD), SN-38, α-amantine, tubulysin analog, cyclophosphamide, mecholrethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, auristatin E, auristatin F and nemorubicin.

11. The modified oligonucleotide-drug conjugate according to claim 8, wherein the drug and at least one of the 5' and 3' ends of the modified oligonucleotide represented by Formula 2 above are conjugated by a linker L in which L₁ and L₂ are bound;
the L₁ is selected from the group consisting of (acrylamide)-C2-NH₂, C12-NH₂, C3-NH₂, acrylamide-C6-propanamide-SH, acrylamide-C6-NH₂, C6-NH₂, and C6-SH, which are selected from the group consisting of 5'-thiol-modifier C6, thiol-modifier C6 S-S, dithiol serinol, PC amino-modifier, 5'-amino-modifier C3, 5'-amino-modifier C6, 5'-amino-modifier C12, 5'-amino-modifier TEG, amino-modifier C2 dT, amino-modifier C6 dT, S-Bz-thiol-modifier C6-dT, phosphodiester bond and nucleotide, and then obtained through a deprotection process; and
the L₂ is selected from the group consisting of maleimidocaproyl-valine-citrulline-p-aminobenzoyloxycarbonyl (MC-Val-Cit-PAB), succinimidyl 4-(N-maleimidomethyl) cyclohexane-1-carboxylate (SMCC), succinic acid, hydrazone, peptide, disulfide, thioether, valine-citrulline, *N*-maleimidomethylcyclohexane-1-carboxylate (MCC), maleimidocaproyl, mercaptoacetamidocaproyl, *N*-succinimidyl 4-(2-pyridyldithio) pentanoate (SPP), *N*-succinimidyl 4-(2-pyridylthio) pentanoate (SPDB), phosphodiester bond and nucleotide.

12. The modified oligonucleotide-drug conjugate according to claim 8, wherein the modified oligonucleotide consists of a sequence selected from SEQ ID NO: 11 or SEQ ID NO: 12.

13. A pharmaceutical composition for preventing or treating cancer comprising the modified oligonucleotide-drug conjugate according to any one of claims 8 to 12.
